# EUROPÄISCHE PATENTSCHRIFT

(11) **EP 2 348 881 B1**
(45) Veröffentlichungstag und Bekanntmachung des Hinweises auf die Patenterteilung: **04.09.2013**
(21) Anmeldenummer: 09737390.6
(22) Anmeldetag: 09.10.2009
(51) Int. Cl.: A23K 1/16, A23K 1/18, C07C 319/20, C07D 241/08, C07C 319/28

(54) **HERSTELLUNG UND VERWENDUNG VON METHIONYLMETHIONIN ALS FUTTERMITTELADDITIV FÜR FISCHE UND KRUSTENTIERE**
PRODUCTION AND USE OF METHIONYL-METHIONINE AS A FEED ADDITIVE FOR FISH AND CRUSTACEANS
PRÉPARATION ET UTILISATION DE MÉTHIONYLMÉTHIONINE EN TANT QU'ADDITIF AUX PRODUITS D'ALIMENTATION ANIMALE, POUR DES POISSONS ET DES CRUSTACÉS

(30) Priorität: 17.10.2008 DE 102008042932
(43) Veröffentlichungstag der Anmeldung: 03.08.2011
(73) Patentinhaber: Evonik Degussa GmbH, 45128 Essen (DE)
(72) Erfinder: KOBLER, Christoph, 63755 Alzenau (DE); HÄUSSNER, Thomas, 63619 Bad Orb (DE); WECKBECKER, Christoph, 63584 Gründau-Lieblos (DE)
(86) Internationale Anmeldenummer: PCT/EP2009/063160
(87) Internationale Veröffentlichungsnummer: WO 2010/043558

(56) Entgegenhaltungen:
- EP-A- 1 564 208
- EP-A- 1 760 074
- US-A- 3 980 653
- US-A- 4 056 658
- BAKER D H; BAFUNDO K W; BOEBEL K P; CZARNECKI G L; HALPIN K M: "METHIONINE PEPTIDES AS POTENTIAL FOOD SUPPLEMENTS EFFICACY AND SUSCEPTIBILITY TO MAILLARD BROWNING" JOURNAL OF NUTRITION, Bd. 114, Nr. 2, 1984, Seiten 292-297, XP002564018
- FRIEDMAN M; GUMBMANN M R: "NUTRITIONAL VALUE AND SAFETY OF METHIONINE DERIVATIVES ISOMERIC DIPEPTIDES AND HYDROXY ANALOGS IN MICE" JOURNAL OF NUTRITION, Bd. 118, Nr. 3, 1988, Seiten 388-397, XP002564023

## Beschreibung

### Einleitung

Die vorliegende Erfindung betrifft neue chemische Synthesen von Methionylmethionin, das Dipeptid von Methionin, und dessen spezielle Verwendung als Futtermitteladditiv allein oder in Mischung mit Methionin für die Ernährung von Fischen und Krustentieren.

### Stand der Technik

Essentielle Aminosäuren (EAA) wie Methionin, Lysin oder Threonin sind als Futtermitteladditive sehr wichtige Bestandteile der Tierernährung und spielen bei der wirtschaftlichen Aufzucht von Nutztieren wie z.B. Hühner, Schweinen und Wiederkäuern eine bedeutende Rolle. Die Supplementierung von natürlichen Eiweißquellen wie z.B. Soja, Mais und Weizen mit EAAs ermöglicht zum einen ein schnelleres Wachstum der Tiere bzw. eine höhere Milchproduktion bei Hochleistungsmilchkühen, zum anderen aber auch die effizientere Verwertung des Futters. Dies stellt einen sehr großen wirtschaftlichen Vorteil dar. Die Märkte für Futtermitteladditive sind von großer industrieller und wirtschaftlicher Bedeutung. Zudem sind sie starke Wachstumsmärkte, was nicht zuletzt auf die steigende Bedeutung von Ländern wie beispielsweise China und Indien zurückzuführen ist.

L-Methionin ((S)-2-Amino-4-methylthiobuttersäure) stellt für viele Tierarten wie Hühner, Enten, Puten und auch für viele Fisch- und Schalentierarten die erste limitierende Aminosäure dar und spielt daher in der Tierernährung und als Futtermitteladditiv eine sehr bedeutende Rolle (Rosenberg et al., J. Agr. Food Chem. 1957, 5, 694-700 und Lovell, T. R., J. Anim. Sci. 1991, 69, 4193-4200). Bei der klassischen chemischen Synthese fällt Methionin jedoch als Racemat, eine 50:50-Mischung aus D- und L-Methionin, an. Dieses racemische DL-Methionin kann jedoch direkt als Futtermitteladditiv eingesetzt werden, da bei einigen Tierarten unter in vivo-Bedingungen ein Umwandlungsmechanismus besteht, der das unnatürliche D-Enantiomer von Methionin in das natürliche L-Enantiomer überführt. Dabei wird das D-Methionin zuerst mit Hilfe einer unspezifischen D-Oxidase zu α-Keto-Methionin desaminiert und anschließend mit einer L-Transaminase zu L-Methionin weiter umgewandelt (Baker, D.H. in "Amino acids in farm animal nutrition", D'Mello, J.P.F. (ed.), Wallingford (UK), CAB International, 1994, 37-61). Dadurch wird die verfügbare Menge an L-Methionin im Organismus erhöht, die dann dem Tier zum Wachstum zur Verfügung stehen kann. Die enzymatische Umwandlung von D- zu L-Methionin wurde bei Hühnern, Schweinen und Kühen, insbesondere aber auch bei carni- und omnivoren Fischen und auch bei Shrimps und Prawns festgestellt. So konnte beispielsweise Sveier et al. (Aquacult. Nutr. 2001, 7 (3), 169-181) und Kim et al. (Aquaculture 1992, 101 (1-2), 95-103) zeigen, dass die Umwandlung von D- in L-Methionin bei karnivoren atlantischen Lachsen und Regenbogenforellen möglich ist. Gleiches konnten Robinson et al. (J. Nutr. 1978, 108 (12), 1932-1936) und Schwarz et al. (Aquaculture 1998, 161, 121-129) für omnivore Fischarten wie zum Beispiel Catfish und Karpfen zeigen. Darüber hinaus konnten Forster und Dominy (J. World Aquacult. Soc. 2006, 37 (4), 474-480) in Fütterungsversuchen von omnivoren Shrimps der Art *Litopenaeus vannamei* zeigen, dass DL-Methionin die gleiche Wirksamkeit wie L-Methionin besitzt.

2007 wurden weltweit über 700.000 t kristallines DL-Methionin bzw. racemisches, flüssiges Methionin-Hydroxy-Analog (MHA, *rac*-2-Hydroxy-4-(methylthio)butansäure (HMB)) und festes Calcium-MHA produziert und erfolgreich bei monogastrischen Tieren wie z.B. Geflügel und Schweinen direkt als Futtermitteladditiv eingesetzt. Durch die rasante wirtschaftliche Entwicklung der Fisch- und Krustentierzucht in hoch industrialisierten Aquakulturen gewinnt eine optimale, wirtschaftliche und effiziente Supplementierungsmöglichkeit von Methionin gerade in diesem Bereich in den letzten Jahren eine immer bedeutendere Rolle (Food and Agriculture Organization of the United Nation (FAO) Fisheries Department "State of World Aquaculture 2006", 2006, Rome. International Food Policy Research Institute (IFPRI) "Fish 2020: Supply and Demand in Changing Markets", 2003, Washington, D.C.). Dabei kann es im Gegensatz zu Hühnern und Schweinen bei der Verwendung von Methionin, MHA oder Ca-MHA als Futtermitteladditiv jedoch bei bestimmten Fisch- und Krustentiersorten zu unterschiedlichen Problemen kommen. So berichten Rumsey und Ketola (J. Fish. Res. Bd. Can. 1975, 32, 422-426), dass die Verwendung von Sojamehl in Verbindung mit einzeln supplementierten, kristallinen Aminosäuren zu keiner Wachstumssteigung bei Regenbogenforellen führte. Murai et al. (Bull. Japan. Soc. Sci. Fish. 1984, 50 (11), 1957) konnte zeigen, dass die tägliche Fütterung von Fischdiäten mit hohen Raten an supplementierten kristallinen Aminosäuren bei Karpfen dazu führten, dass über 40% der freien Aminosäuren über die Kiemen und Nieren ausgeschieden werden. Aufgrund der schnellen Resorption von supplementierten Aminosäuren kurz nach der Futteraufnahme, kommt es zu einem sehr schnellen Anstieg der Aminosäurenkonzentration im Blutplasma des Fisches (Fast-Response). Zu dieser Zeit befinden sich aber die anderen Aminosäuren aus den natürlichen Proteinquellen wie z.B. Sojamehl noch nicht im Plasma, was zur Asynchronität der gleichzeitigen Verfügbarkeit aller wichtigen Aminosäuren führen kann. Ein Teil der hochkonzentrierten Aminosäuren wird in Folge dessen schnell ausgeschieden bzw. im Organismus schnell metabolisiert und z.B. als reine Energiequelle genutzt. Dadurch kommt es beim Karpfen nur zu einer geringen bzw. keiner Wachstumssteigerung bei der Verwendung von kristallinen Aminosäuren als Futtermitteladditive (Aoe et al., Bull. Jap. Soc. Sci. Fish. 1970, 36, 407-413). Bei Krustentieren kann die Supplementierung von kristallinen Aminosäuren noch zu weiteren Problemen führen. Durch das langsame Fressverhalten von bestimmten Krustentieren wie z.B. Shrimps der Art *Litopenaeus Vannamei* kommt es, aufgrund der langen Verweilzeit des Futters unter Wasser, zum Herauslösen der supplementierten, wasserlöslichen Aminosäuren (Leaching), was zur Eutrophierung des Gewässers und nicht zu einer Wachstumssteigerung der Tiere führt (Alam et al., Aquaculture 2005, 248, 13-16).

Die effektive Versorgung von Fischen und Krustentieren, die in Aquakulturen gehalten werden, erfordert somit für bestimmte Arten und Anwendungen eine spezielle Produktform von Methionin wie z.B. ein entsprechend chemisch oder physikalisch geschütztes Methionin. Dabei ist das Ziel zum einen, dass das Produkt während der Fütterung in der wässrigen Umgebung hinreichend stabil bleibt und nicht aus dem Futter herausgelöst wird. Zum anderen, dass das schließlich vom Tier aufgenommene Methioninprodukt im tierischen Organismus optimal und mit hoher Effizienz verwertet werden kann.

In der Vergangenheit wurden viele Anstrengungen unternommen, um geeignete Futtermitteladditive, im Besonderen auf Methioninbasis, für Fische und Krustentiere zu entwickeln. Sö wird beispielsweise in WO8906497 die Verwendung von Di- und Tripeptiden als Futtermitteladditiv für Fisch- und Krustentieren beschrieben. Dadurch soll das das Wachstum der Tiere gefördert werden. Dabei kamen aber bevorzugt Di- und Tripeptide aus nicht essentiellen und daher auch nicht limitierenden Aminosäuren wie z.B. Glycin, Alanin und Serin zum Einsatz. Als Methionin haltige Dipeptide wurde nur DL-Alanyl-DL-Methionin und DL-Methionyl-DL-Glycin beschrieben. Dadurch sind im Dipeptid aber nur effektiv 50% Wirkstoff (mol/mol) enthalten, was unter wirtschaftlichen Gesichtspunkten als sehr nachteilig einzustufen ist. In W002088667 wird die enantioselektive Synthese und Anwendung von Oligomeren aus MHA und Aminosäuren wie z.B. Methionin als Futtermitteladditive, unter anderem auch für Fische und Krustentiere, beschrieben. Dadurch soll ein schnelleres Wachstum erzielt werden können. Die beschriebenen Oligomere werden durch eine enzymkatalysierte Reaktion aufgebaut und weisen eine sehr breite Verteilung der Kettenlänge der einzelnen Oligomere auf. Dadurch wird das Verfahren unselektiv, teuer und aufwendig in der Durchführung und Aufreinigung. Dabrowski et al. beschreibt in US20030099689 die Verwendung von synthetischen Peptiden als Futtermitteladditive zur Wachstumsförderung für aquatische Tiere. Dabei können die Peptide einen Gewichtsanteil von 6-50% der gesamten Futterformulierung ausmachen. Die synthetischen Peptide bestehen bevorzugt aus essentiellen und limitierenden Aminosäuren. Die Synthese solcher synthetischer Oligo- und Polypeptide ist jedoch sehr aufwendig, teuer und großtechnisch schwer umsetzbar. Zudem ist die Wirksamkeit von Polypeptiden einer einzelnen Aminosäure umstritten, da diese oft nur sehr langsam oder gar nicht unter physiologischen Bedingungen zu freien Aminosäuren umgesetzt werden. So beschreibt beispielsweise Baker et al. (J. Nutr. 1982, 112, 1130-1132), dass Poly-L-Methionin aufgrund der absoluten Unlöslichkeit in Wasser keine Biowertigkeit bei Hühnern aufweist, da eine Resorption vom Organismus nicht möglich ist.

Neben dem Einsatz neuer chemischer Methioninderivate wie z.B. methioninhaltige Peptide und Oligomere wurden auch verschiedene physikalische Schutzmöglichkeiten wie z.B Coatings bzw. die Einbettung einer Aminosäure in einer Schutzmatrix untersucht. So konnten beispielsweise Alam et al. (Aquacult. Nutr. 2004, 10, 309-316 und Aquaculture 2005, 248, 13-19) zeigen, dass gecoatetes Methionin und Lysin im Gegensatz zu nicht gecoatetem einen sehr positiven Einfluss auf das Wachstum von jungen Kuruma Shrimps besitzt. Obwohl die Verwendung eines speziellen Coatings das Leaching von Methionin und Lysin aus dem Futterpellet unterdrücken konnte, gibt es einige gravierende Nachteile. Die Herstellung bzw. die Beschichtung von Methionin stellt meist ein technisch kompliziertes und aufwendiges Verfahren dar und ist daher teuer. Zudem kann die oberflächliche Beschichtung des fertig gecoateten Methionins leicht durch mechanische Belastung und Abrieb während der Futterverarbeitung beschädigt werden, was zur Verminderung bzw. bis zum vollständigen Verlust des physikalischen Schutzes führen kann. Hinzukommt, dass durch ein Coating oder Verwendung einer Matrixsubstanz der Gehalt an Methionin verringert und damit oft unwirtschaftlich wird.

Neben der erfinderischen neuen Anwendung von DL-Methionyl-DL-methionin als Futtermitteladditiv mit geringem Leachingverhalten aus Futterpellets und -extrudaten, sowie einer optimalen Methioninversorgung im Organismus durch "Slow Release" Spaltung von Methionylmethionin, konnten auch neue Verfahren zur Darstellung von Methionylmethionin entwickelt werden, die viele Vorteile gegenüber der in der Literatur beschriebenen Darstellungsvarianten besitzen. Die meisten literaturbekannten Synthesen von Dipeptiden verwenden teure Schutzgruppen wie z.B. Boc- (tert-Butoxycarbonyl-) oder Z-(Benzyloxycarbonyl-) Schutzgruppen, die vor der eigentlichen Dipeptidsynthese an die entsprechende Aminosäure angebracht und später wieder abgespalten werden müssen. Zudem ist meist eine Aktivierung der zu koppelnden Aminosäuren nötig. So lässt sich Methionylmethionin durch Kopplung von N-Boc-Methionin mit dem Methylester von Methionin mit Hilfe von Dicyclohexylcarbodiimid (DCC) darstellen. Die großen Nachteile dieses Darstellungsverfahren sind die Verwendung von teuren Schutzgruppen, großer Syntheseaufwand und nicht recyclierbare und teure Kopplungsreagentien wie z.B. DCC. Eine andere Alternative zur technischen Synthese von Methionylmethionin wird in DE2261926 beschrieben. Durch Erhitzen des Isopropylesters von Methionin wird in der ersten Stufe das 3,6-Bis[2-(methylthio)ethyl]-2,5-piperazindion (Methionindiketopiperazin, DKP) gebildet, welches anschließend zu Methionylmethionin verseift werden kann. Dabei konnten für den Verseifungsschritt lediglich befriedigende Ausbeuten von 62-65% erzielt werden. Zudem ist die Verwendung von Methioninisopropylester als Ausgangsmaterial zu teuer und daher unwirtschaftlich.

Baker, D. H. et al. (Journal of Nutrition, Band 114, Nr. 2, 1984, Seite 292-297) offenbart (Seite 293, linke Spalte) auch ein Verfahren zur Herstellung von DL-methionyl-DL-methionin aus einem Diketopiperazin, welches zuvor aus DL-Methioninisopropylester hergestellt worden ist.

EP1760074 und EP1564208 offenbarten jeweils ein Verfahren zur Herstellung von DL-Methionin unter Umsetzung von Methioninhydantoin (Formel (IId) in der Beschreibung) bei dem auch D,L-Methionyl-DL-methionin als Nebenprodukt entsteht, welches durch Kristallisation abgetrennt wird bzw. durch Hydrolyse zu Methionin gespalten wird.

US3980653 offenbart ein Verfahren zur Herstellung von 3,6-bis- (2-methylmercaptoethyl)-2,5-piperazindion (Methionindiketopiperazin) unter Umsetzung von Methioninhydantoin mit Methionin bei 160°C und einem Druck von zunächst 9.5 bar. Das durch Kristallisation isolierte Diketopiperazin ist zum Beispiel zu Methionyl-methionin weiter umsetzbar.

US4056658 offenbart die Verwendung von DL-Methionyl-DL-methionin als Futtermitteladditiv in Futtermittelmischungen enthaltend Sojaproteine für Landtiere wie Rindvieh oder Schafe, sowie ein Fütterungsexperiment an Albino-Ratten.

### Aufgabe der Erfindung

Eine generelle Aufgabe war es, ein Futtermittel bzw. einen Futtermittelzusatzstoff für die Tierernährung auf Basis eines neuen Methioninersatzstoffes bereitzustellen, der alleine oder als Mischung mit Methionin besonders im Bereich der industriellen Fisch- und Krustentierzucht in Aquakulturen eingesetzt werden kann. Gleichzeitig sollte eine einfache und kostengünstige chemische Synthese dieses neuen Methioninersatzstoffes entwickelt werden.

Vor dem Hintergrund der Nachteile des Standes der Technik war es vor allem die Aufgabe, ein chemisch geschütztes Methioninprodukt für verschiedene omni-, herbi- und karnivore Fisch- und Krustentierarten, die in Salz- oder Süßwasser leben, bereitzustellen. Insbesondere sollte dieses Produkt ein geringes Löslichkeitsverhalten (Leaching) aus dem Gesamtfutterpellet bzw. -extrudat im Wasser aufweisen und einen "Slow Release"-Mechanismus, also eine langsame und kontinuierliche Freisetzung von freiem Methionin unter physiologischen Bedingungen besitzen. Außerdem sollte das neue Methioninprodukt auch als Mischung mit DL-Methionin vorteilhaft eingesetzt werden können.

Eine weitere Aufgabe war es, ein Methioninersatzstoff als Futtermittel bzw. einen Futtermittelzusatzstoff mit sehr hoher Biowertigkeit aufzufinden, der gute Handhabbarkeit und Lagerfähigkeit sowie Stabilität unter den üblichen Be-dingungen der Mischfutterverarbeitung, insbesondere der Pelletierung und Extrusion aufweisen sollte.

Auf diese Weise sollte den Fischen und Krustentieren neben kristallinem DL-Methionin eine weitere effiziente Methioninquelle zur Verfügung gestellt werden, welche möglichst die Nachteile der bekannten Produkte nicht oder nur in verringertem Umfang aufweist.

Des Weiteren sollte eine neue, flexible Syntheseroute für Methionylmethionin (DL-Methionyl-DL-methionin) entwickelt werden, bei der typische Vor- und Nebenprodukten aus dem technischen DL-Methionin Produktionsprozess als Startmaterial verwendet werden können. Außerdem sollte ein geeignetes Verfahren zur Trennung der Diastereomerenpaare DD/LL- und DL/LD-Methionylmethionin entwickelt werden, damit für spezielle Anwendungen auch ein optimaler und effektiver Einsatz von nur einem Diastereomerenpaar (DD/LL-I oder DL/LD-I) möglich ist.

### Beschreibung der Erfindung

Die Aufgabe wird gelöst durch die Verwendung von DL-Methionyl-DL-methionin und dessen Salze als Futtermitteladditiv in Futtermittelmischungen für in Aquakulturen gehaltene Tiere.

In bevorzugter Weise enthält die Futtermittelmischung 0,01 bis 5 Gew.-%, bevorzugt 0,05 bis 0,5 Gew.-% DL-Methionyl-DL-methionin enthält.

Die Verwendung von DL-Methionyl-DL-methionin hat sich hierbei als besonders vorteilhaft erwiesen, weil die Verbindung ein hervorragendes Leaching-Verhalten aufgrund der niedrigen Löslichkeit der Mischung aus DD/LL/DL/LD-Methionylmethionin bzw. des Diastereomerenpaars DL/LD-Methionylmethionin (0,4 g/l) aufweist.

Weiterhin zeigt die Verbindung eine gute Pelletier- und Extrusionsstabilität bei der Futtermittelherstellung. DL-Methionyl-DL-methionin ist stabil in Mischungen mit üblichen Komponenten und Futtermitteln wie z.B. Getreide (z.B. Mais, Weizen, Tritikale, Gerste, Hirse, u.a.), pflanzliche oder tierische Eiweißträger (z.B. Sojabohnen und Raps und deren Weiterverarbeitungsprodukte, Leguminosen (z.B. Erbsen, Bohnen, Lupinen, etc.), Fischmehl, u.a.) und in Kombination mit supplementierten essentiellen Aminosäuren, Proteinen, Peptiden, Kohlenhydraten, Vitaminen, Mineralien, Fetten und Ölen.

Weiterhin ist von Vorteil, dass durch den hohen Wirkstoffanteil von Methionylmethionin pro kg Substanz, im Vergleich zu DL-Methionin pro Mol Methionylmethionin ein Mol Wasser eingespart wird.

In einer bevorzugten Verwendung enthält die Futtermittelmischung Proteine und Kohlehydrate, vorzugsweise auf Basis von Fisch-, Soja- oder Maismehl, und können mit essentiellen Aminosäuren, Proteinen, Peptiden, Vitaminen, Mineralien, Kohlenhydraten, Fetten und Ölen supplementiert sein.

Insbesondere ist bevorzugt, dass in der Futtermittelmischung DL-Methionyl-DL-methionin allein als DD/LL/LD/DL-Mischung, als DL/LD- oder DD/LL-Mischung vorliegt, bevorzugt jeweils zusätzlich in Mischung mit DL-Methionin, vorzugsweise mit einem DL-Methioninanteil von 0,01 bis 20 Gew.-%, besonders bevorzugt von 1 bis 10 Gew.-%.

In einer besonders bevorzugten Verwendung liegt DL-Methionyl-DL-methionin als Enantiomerenpaar DL/LD-Methionylmethionin vor.

In einer bevorzugten Verwendung sind die in Aquakulturen gehaltene Tiere Süß- und Salzwasserfische und -krustentiere ausgewählt aus der Gruppe bestehend aus Karpfen, Forellen, Lachse, Welse, Barsche, Plattfische, Störe, Thunfische, Aale, Brassen, Dorsche, Shrimps, Krill und Prawns, ganz besonders für Silver- (*Hypophthalmichthys molitrix*), Gras-(*Ctenopharyngodon idella*), Schuppen- (*Cyprinus carpio*) und Bigheadkarpfen (*Aristichthys nobilis*), Karausche (*Carassius carassius*), Catla (*Catla Catla*), Roho Labeo (*Labeo rohita*), pazifischer und atlantischer Lachs (*Salmon salar* und *Oncorhynchus kisutch*), Regenbogenforelle (*Oncorhynchus mykiss*), amerikanischer Wels (*Ictalurus punctatus*), afrikanischer Wels (*Clarias gariepinus*), Pangasius (*Pangasius bocourti* und *Pangasius hypothalamus),* Niltilapie (*Oreochromis niloticus),* Milchfisch (*Chanos chanos),* Cobia (*Rachycentron canadum*)*,* Whiteleg Shrimp (*Litopenaeus vannamei),* Black Tiger Shrimp (*Penaeus monodon)* und Giant River Prawn (*Macrobrachium rosenbergii*).

Gemäß der Erfindung wird DL-Methionyl-DL-methionin (**I**) (kurz Methionylmethionin oder Met-Met) oder dessen Alkali- und Erdalkalisalze wie z.B. das schwerlösliche Calcium- oder Zinksalz als Zusatz in Futtermittelgemischen als DD/LL/DL/LD-, DD/LL- oder DL/LD-Diastereomerengemisch, alleine oder in Mischung mit DL-Methionin, vorzugsweise für Fische und Krustentiere, verwendet:

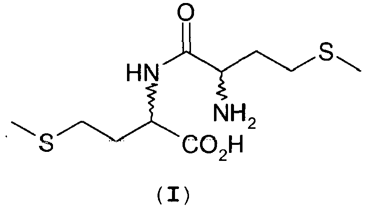

Vom Dipeptid DL-Methionyl-DL-methionin (**I**) existieren vier verschiedene Stereoisomere (Diastereomere) DD-, LL, DL- und LD-**I,** wovon nur das L-Methionyl-L-methionin (LL-**I**) natürlich ist, die drei anderen Dipeptide L-Methionyl-D-methionion (LD-**I**), D-Methionyl-L-methionin (DL-**I**) und D-Methionyl-D-methionin (DD-**I**) alle nichtnatürlich sind (siehe Schema 1).

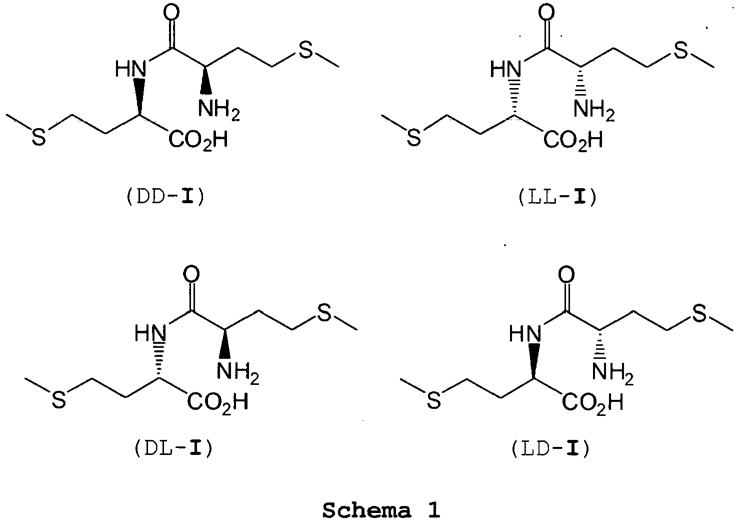

Dabei verhalten sich DD-**I** und LL-**I** untereinander wie Bild und Spiegelbild, d.h. es sind Enantiomere und besitzen damit auch die gleichen physikalischen Eigenschaften. Gleiches gilt für das Paar DL-**I** und LD-I**.**

Die beiden Paare DD/LL-**I** und DL/LD-**I** sind dagegen diastereomer zueinander, d.h. sind haben unterschiedliche physikalische Daten. So besitzt beispielsweise das Diastereomerenpaar DD/LL-**I** bei Raumtemperatur eine Löslichkeit von 21,0 g/l in Wasser, wohingegen die Löslichkeit vom Diastereomerenpaar DL/LD-**I** bei 0,4 g/l liegt.

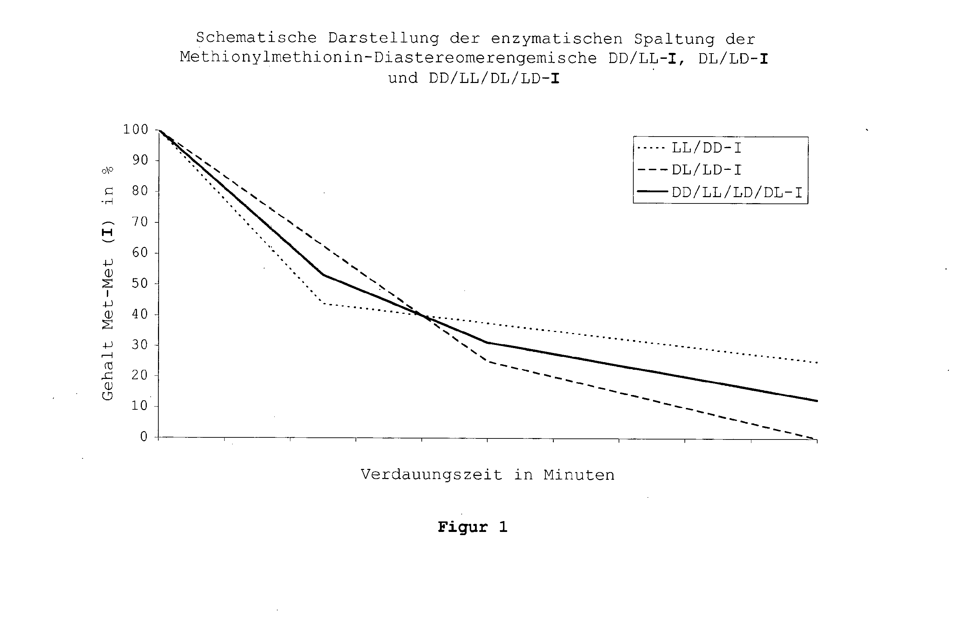

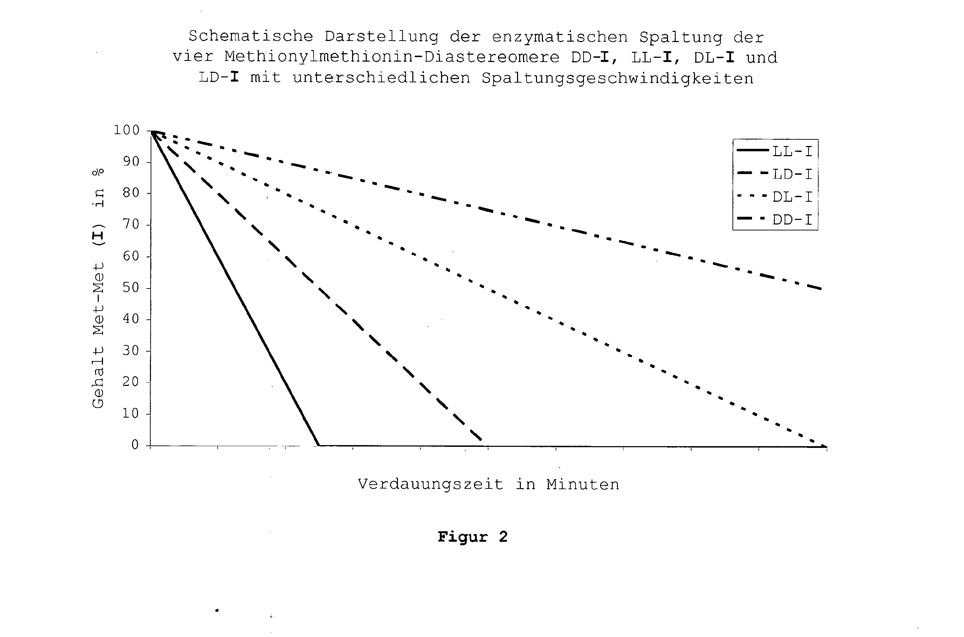

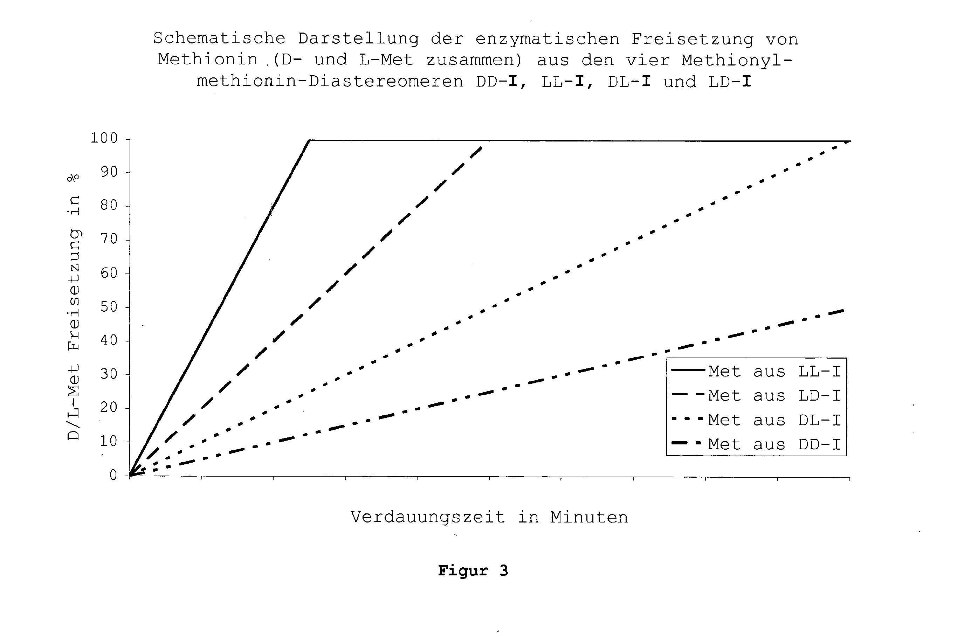

Neben der Entwicklung neuer Synthesenmethoden für Methionylmethionin ist der Gegenstand der vorliegenden Erfindung die Verwendung von DL-Methionyl-DL-methionin als Futtermittel als DD/LL/DL/LD-, DD/LL- oder DL/LD-Diastereomerengemisch als Wachstumspromotor für omni-, carni- und herbivore Fische und Krustentiere in Aquakulturen. So konnte erfinderisch gezeigt werden, dass DL-Methionyl-DL-methionin (**I**) unter physiologischen Bedingungen enzymatisch von Fischen und Krustentieren zu freiem D- bzw. L-Methionin gespalten werden kann (Schema 2) (siehe auch Beispiele 22 bis 24). Dazu wurden die entsprechenden Verdauungsenzyme aus Karpfen (omnivor), Forelle (karnivor) und Whiteleg Shrimp (omnivor) isoliert und in optimierten *in vitro* Versuchen unter physiologisch vergleichbaren Bedingungen mit DL-Methionyl-DL-methionin umgesetzt. Die erfindungsgemäße Besonderheit der Spaltung von DL-Methionyl-DL-methionin (**I**) liegt darin, dass alle vier möglichen Diastereomere, sowohl das natürliche LL-**I,** als auch die drei unnatürlichen Diastereomere DD-, DL- und LD-**I** unter physiologischen Bedingungen gespalten werden können. Dies gilt sowohl für die Verwendung der Gesamtmischung aller Diastereomere (DD/LL/DL/LD-**I**), als auch jeweils für die beiden Diastereomerenpaare DD/LL-**I** bzw. DL/LD-**I** (siehe Figur 1). 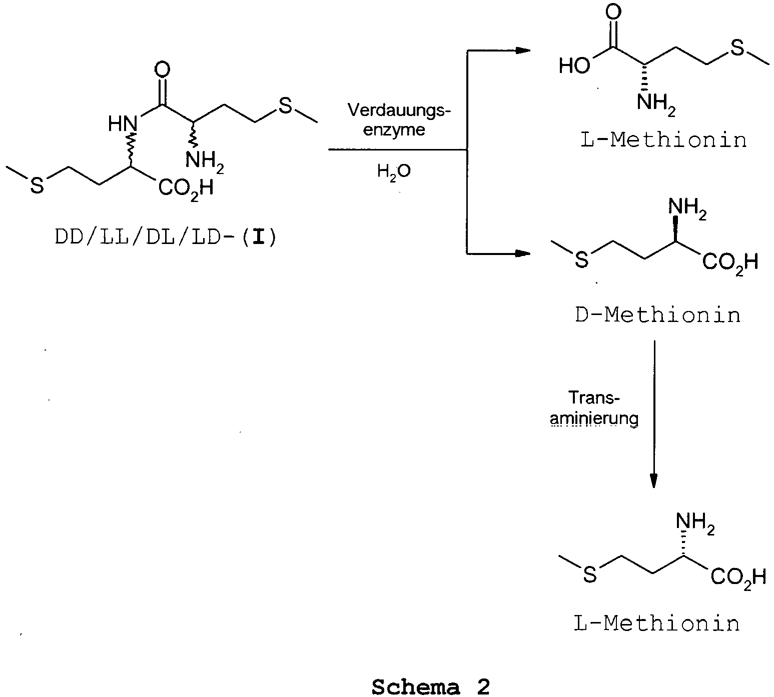 Die Spaltung der einzelnen Diastereomere von Methionylmethionin geschieht jedoch mit unterschiedlichen Geschwindigkeiten. Dies verdeutlicht die schematische Darstellung der enzymatischen Spaltung der einzelnen Diastereomere von Methionylmethionin mit Verdauungsenzymen von Fischen und Krustentieren in Figur 2. Durch die verzögerte Spaltung wird damit aber auch die Freisetzung von D- und L-Methionin ebenfalls verzögert (siehe Figur 3). Dies hat den großen Vorteil, dass es zu keiner "Fast-Response"-Resorption von freiem D- bzw. L-Methionin im Verdauungstrakt und damit auch nicht zu einer Konzentrationsspitze von freiem Methionin im Blutplasma kommen kann.

Der Vorteil bei der Verwendung von Methionylmethionin als Futtermitteladditiv und Methioninquelle ist damit, dass D-bzw. L-Methionin über die gesamte Verdauungszeit im Organismus freigesetzt werden kann und damit synchron mit der Freisetzung anderer, aus natürlichen Proteinquellen stammender Aminosäuren verläuft ("Slow-Release"-Mechanismus) (siehe Figur 3). Dieser besondere Effekt hat zur Folge, dass die gleichzeitige Verfügbarkeit aller wichtigen und essentiellen Aminosäuren in einem idealen Verhältnis im Blutplasma sichergestellt ist, was für ein optimales Wachstum des Organismus absolut nötig ist.

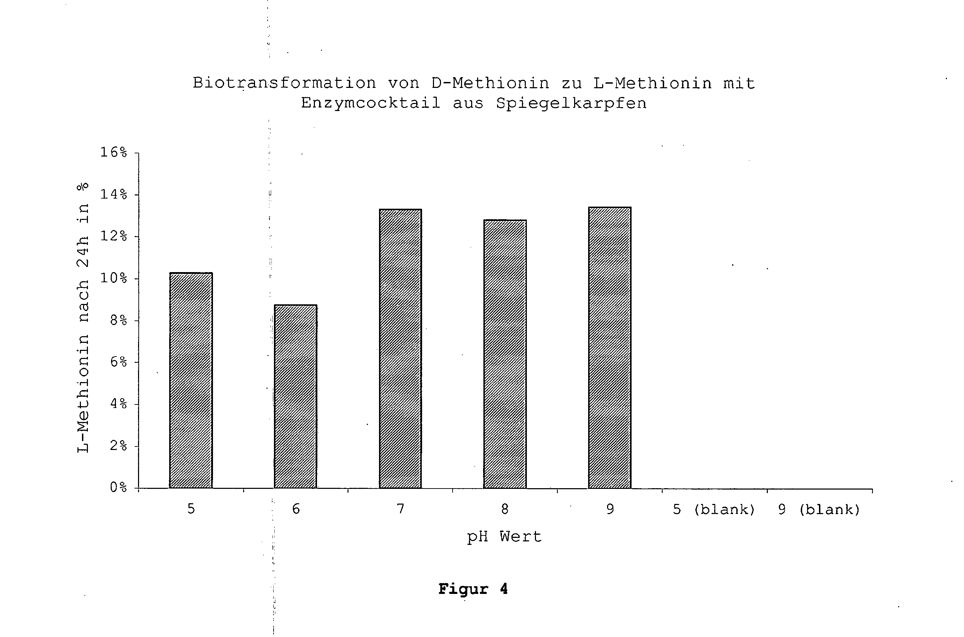

Bei der enzymatischen Dipeptidspaltung von DL-Methionyl-DL-methionin (**I**) wird neben dem natürlichen L-Methionin auch das unnatürliche D-Methionin freigesetzt (siehe Schema 2). Dieses kann sowohl von karni-, omni- und herbivoren Salz- bzw. Süßwasserfischen und -krustentieren enzymatisch zum natürlichen L-Methionin transaminiert werden. Dies konnte in Beispiel 25 am Beispiel von Karpfen gezeigt werden. Mit Hilfe eines Enzymcocktail aus Verdauungs- und Leberenzymen aus Karpfen konnte D- in L-Methionin unter physiologisch entsprechenden Bedingungen umgewandelt werden (siehe Figur 4). Damit ist eine optimale Versorgung des Organismus von natürlichem L-Methionin bei der Verwendung von DL-Methionyl-DL-methionin (**I**) gewährleistet.

Die Pelletier- und Extrusionsversuche mit verschiedenen Mischungen aus DL-Methionyl-DL-methionin (**I**) und natürlichen Protein- und Kohlehydratquellen wie beispielsweise Fisch-, Mais- und Sojamehl sowie in Mischungen mit anderen essentiellen Aminosäuren, Proteinen, Peptiden, Vitaminen, Mineralien, Fetten und Ölen zeigen, dass DL-Methionyl-DL-methionin (**I**) während und nach dem Produktionsprozess absolut stabil ist und es zu keinerlei Abbau oder Zersetzung kommt (siehe Beispiel 26).

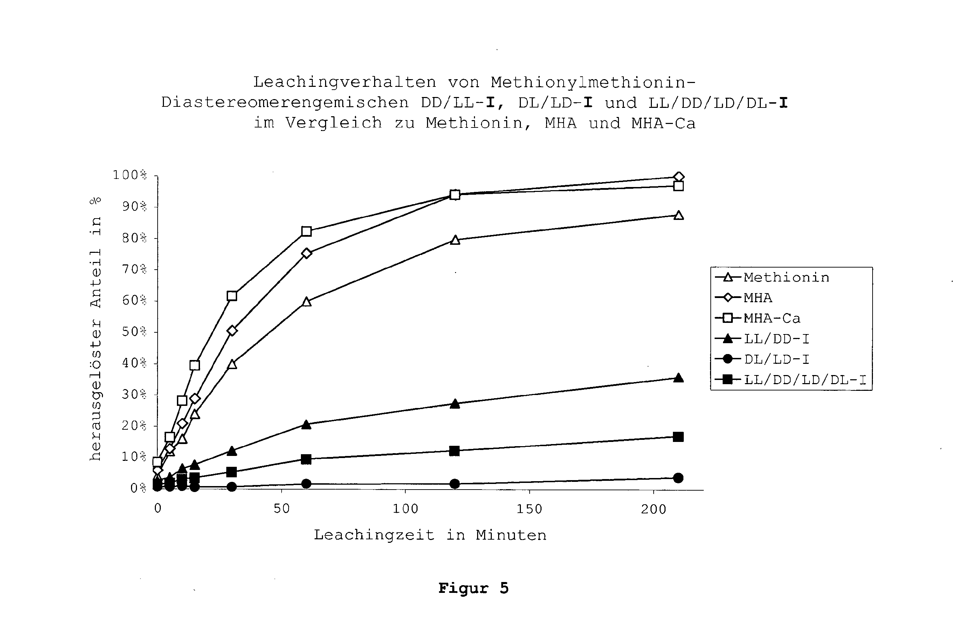

Um das Leachingverhalten der Diastereomere von Methionylmethionin (**I**) aus Mischfutterpellets unter Wasser zu untersuchen, wurde die zeitliche Abhängigkeit des Herauslösens von Methionylmethionin gemessen (siehe Beispiel 26). Als Vergleich wurde das Leachingverhalten von DL-Methionin, MHA und Calcium-MHA (MHA-Ca) unter den identischen Bedingungen untersucht. Dabei zeigte sich, dass sowohl die Gesamtmischung aller Diastereomere (DD/LL/DL/LD-**I**), als auch die Diastereomerenpaare DD/LL-**I** und DL/LD-**I** ein deutlich niedrigeres Leaching zeigen als DL-Methionin, MHA und Calcium-MHA (MHA-Ca) (siehe Figur 5). Damit wird über die Zeit viel weniger Methionylmethionin aus den Futterpellets gelöst als bei allen anderen Methioninderivaten. Besonders niedrige Leachingraten zeigt das Diastereomerenpaar DL/LD-**I**, welches auch nach über 200 Min. Verweilzeit nur zu maximal 5% aus den Futterpellets herausgelöst wurde (siehe Figur 5).

Die Aufgabe wird weiterhin gelöst durch ein Verfahren zur Herstellung von DL-Methionyl-DL-methionin (**I**) mit der Formel 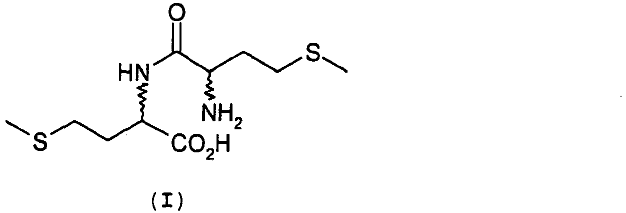 unter Umsetzung eines Harnstoffderivates der allgemeinen Formel **II**, 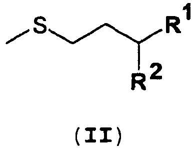 wobei die Reste R¹ und R² in den Harnstoffderivaten **IIa, IIb, IIc, IId, IIe, IIf** und **IIg** wie folgt definiert sind:
wobei
**IIa: R¹ =** COOH, **R²** = NHCONH₂
**IIb: R¹** = CONH₂, **R²** = NHCONH₂
**IIc: R¹ =** CONH₂, **R²** = NH₂
**(IId: R¹-R² =** -CONHCONH-, nicht erfindungsgemäβ)
**IIe : R¹** = CN, **R² =** OH
**IIf: R¹** = CN, **R²** = NH₂
**(IIg: R¹** = =O, **R²** = H, nicht erfindungsgemäβ bedeutet,
bedeutet,
zu DL-Methionyl-DL-methionin (**I**).

In einer Ausführungsform des beschreibungsgemäßen Verfahrens ist dabei bevorzugt, dass Methioninhydantoin (IId) als Ausgangsprodukt verwendet wird oder als Zwischenprodukt intermediär gebildet wird. Bei diesem Verfahren wird DL-Methionyl-DL-methionin direkt aus Methioninhydantoin synthetisiert und umfasst die in Schema 3 aufgezeigten Methoden G, H, und J.

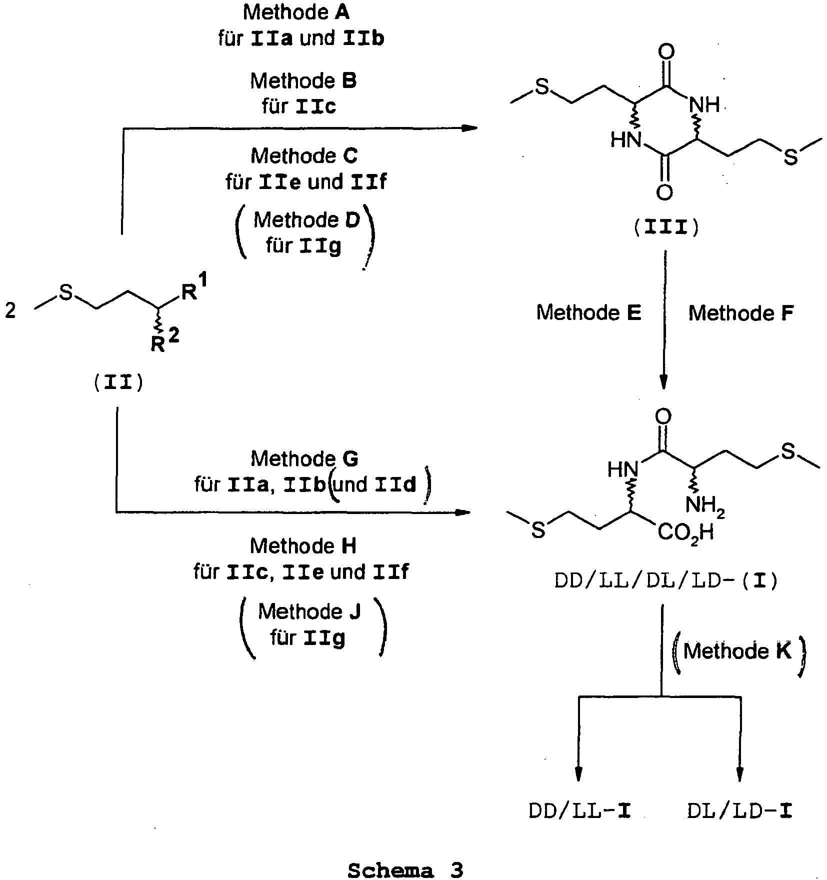

Die in Klammern gesetzten Methoden gehören nicht zur Erfindung.

Bevorzugt, wird dabei eine Methioninhydantoin und Wasser enthaltende Lösung mit Methionin unter basischen Bedingungen umgesetzt wird. Weiterhin ist bevorzugt, dass der pH-Wert der das Harnstoffderivat enthaltenden Lösung auf 8 bis 14, bevorzugt auf 10 bis 13 eingestellt wird.

In bevorzugter Weise erfolgt die Umsetzung bei einer Temperatur von 50 bis 200°C, bevorzugt bei einer Temperatur von 80 bis 170°C und besonders bevorzugt bei einer Temperatur von 130 bis 160 °C.

Weiterhin ist bevorzugt, dass die Umsetzung unter Druck, bevorzugt bei einem Druck von 3 bis 20 bar, besonders bevorzugt bei einem Druck von 6 bis 15 bar durchgeführt wird.

In einem erfindungsgemäβ bevorzugten Verfahren ist eine Methioninhydantoin und Wasser enthaltende Lösung zuvor aus einer oder mehrerer der Verbindungen **IIa, IIb, IIc, IIe** und **IIf** gebildet worden.

In einem weiteren bevorzugten Verfahren wird Methioninhydantoin durch Umsetzung der Verbindung **IIe** oder **IIf** mit einer Stickstoff haltigen Base, NH₄HCO₃, (NH₄)₂CO₃, NH₄OH/CO₂-Mischung oder Carbamatsalzen erhalten.

Die Umsetzung der Verbindung **IIe** wird bevorzugt bei einer Temperatur von 0°C bis 150°C, vorzugsweise 0°C bis 100°C und besonders bevorzugt von 10°C bis 70°C durchgeführt.

In einem weiteren bevorzugten Verfahren wird das Methioninhydantoin durch Umsetzung der Verbindung **IIf** mit CO₂ erhalten. Dabei ist bevorzugt, dass die Umsetzung in Anwesenheit einer Base erfolgt, vorzugsweise ausgewählt aus der Gruppe enthaltend KHCO₃, K₂CO₃, tertiäre Amine oder deren Salze, Alkali- und Erdalkalibasen.

In einem weiter beschriebenen Verfahren wird Methioninhydantoin durch Umsetzung der Verbindung **IIg** mit einer Cyanidionenquelle und einer Base erhalten ausgewählt aus der Gruppe enthaltend Stickstoff haltige Basen, Ammoniumsalze in Gegenwart von CO₂, NH₄HCO₃, (NH₄)₂CO₃, NH₄OH/CO₂-Mischung und Carbamatsalze. Die Umsetzung erfolgt dabei bei einer Temperatur von vorzugsweise -20°C bis 150°C, bevorzugt -10°C bis 100°C und besonders bevorzugt von 0°C bis 70°C.

Eine alternative Ausführungsform des erfindungsgemäßen Verfahrens umfasst die folgenden Schritte:
a) Umsetzung des Harnstoffderivates gemäß den Formeln **IIa, IIb, IIc**, **IIe** und **IIf** zu einem Diketopiperazin der Formel 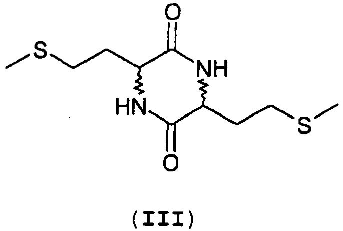
b) Umsetzung des Diketopiperazins zum DL-Methionyl-DL-methionin. Dieses Verfahren umfasst die in Schema 3 aufgezeigten Methoden A, B und C. Bei diesen Verfahren wird Diketopiperazin (**III**) als Zwischenprodukt gebildet.

Dabei ist bevorzugt, dass die Umsetzung des Harnstoffderivates zum Diketopiperazin bei einer Temperatur von 50°C bis 200°C, vorzugsweise von 100°C bis 180°C und besonders bevorzugt von 140°C bis 170°C durchgeführt wird.

In einem bevorzugten Verfahren erfolgt die Umsetzung des Harnstoffderivates zum Diketopiperazin unter Druck, bevorzugt bei einem Druck von 3 bis 20 bar, besonders bevorzugt bei einem Druck von 6 bis 15 bar.

Vorzugsweise erfolgt die Umsetzung des Harnstoffderivates zum Diketopiperazin in Gegenwart einer Base. Dabei ist die

Base vorzugsweise ausgewählt aus der Gruppe Stickstoff haltiger Basen, NH₄HCO₃, (NH₄)₂CO₃, KHCO₃, K₂CO₃, NH₄OH/CO₂-Mischung, Carbamatsalze, Alkali- und Erdalkalibasen.

In einem weiteren bevorzugten Verfahren erfolgt die Umsetzung des Harnstoffderivates zum Diketopiperazin durch Reaktion mit Methionin. Dabei ist ein Verhältnis von Harnstoffderivat zu Methionin von 1:100 bis 1:0,5 bevorzugt.

In einem weiteren bevorzugten Verfahren erfolgt die Umsetzung des Diketopiperazin zum DL-Methionyl-DL-methionin durch saure Hydrolyse. Die saure Hydrolyse wird dabei in Gegenwart einer Säure durchgeführt, die vorzugsweise ausgewählt ist aus der Gruppe der Mineralsäuren, HCl, H₂CO₃, CO₂/H₂O, H₂SO₄, Phosphorsäuren, Carbonsäuren und Hydroxycarbonsäuren.

In einer anderen Ausführungsform des erfindungsgemäßen Verfahrens erfolgt die Umsetzung des Diketopiperazin zum DL-Methionyl-DL-methionin durch basische Hydrolyse. Hierbei wird die basische Hydrolyse bevorzugt bei einem pH von 7 bis 14, besonders bevorzugt bei einem pH von 9 bis 12, ganz besonders bevorzugt bei einem pH von 10 bis 11 durchgeführt, um DL-Methionyl-DL-methionin zu erhalten. Dabei können die basischen Bedingungen unter Verwendung einer Substanz eingestellt werden, die vorzugsweise ausgewählt sind aus der Gruppe Stickstoff haltiger Basen, NH₄HCO₃, (NH₄)₂CO₃, NH₄OH/CO₂-Mischung, Carbamatsalze, KHCO₃, K₂CO₃, Carbonate, Alkali- und Erdalkalibasen.

Die saure bzw. basische Hydrolyse wird vorzugsweise bei Temperaturen von 50°C bis 200°C, vorzugsweise von 80°C bis 180°C und besonders bevorzugt von 90°C bis 160°C durchgeführt.

In einer weiteren Ausführungsform wird die Umsetzung des Diketopiperazin zum DL-Methionyl-DL-methionin durch Einleiten von CO₂ in eine basische Lösung, bevorzugt in eine basische Ammonium-, Kalium- oder Natriumhydroxidlösung durchgeführt.

In einem bevorzugten Verfahren wird das Diketopiperazin vor der Hydrolyse isoliert. Bevorzugt ist dabei, dass das Diketopiperazin durch Kristallisation aus der Reaktionslösung, bevorzugt bei einer Temperatur von -30 bis 120°C, besonders bevorzugt bei einer Temperatur von 10 bis 70°C, isoliert wird.

Zur Isolation des Diastereomerengemisches aus DD/LL/DL/LD-Methionylmethionin aus basischen Reaktionslösungen wird diese angesäuert und das Methionylmethionin durch Kristallisation bzw. Fällung erhalten. Bevorzugt ist dabei ein pH-Wert von 5 bis 9, besonders bevorzugt ein pH-Wert von 5 bis 7, ganz besonders bevorzugt ein pH-Wert von ca. 5,6. Dabei können Säuren bevorzugt aus der Gruppe der Mineralsäuren, HCl, H₂CO₃, CO₂/H₂O, H₂SO₄, Phosphorsäuren, Carbonsäuren und Hydroxycarbonsäuren zur Ansäuerung verwendet werden.

Zur Isolation des Diastereomerengemisches aus DD/LL/DL/LD-Methionylmethionin aus sauren Reaktionslösungen wird durch Zugabe von Basen neutralisiert und das Methionylmethionin durch Kristallisation bzw. Fällung erhalten. Bevorzugt ist dabei ein pH-Wert von 5 bis 9, besonders bevorzugt ein pH-Wert von 5 bis 7, ganz besonders bevorzugt ein pH-Wert von ca. 5,6. Dabei werden zur Neutralisation Basen bevorzugt aus der Gruppe NH₄HCO₃, (NH₄)₂CO₃, Stickstoff haltige Basen, NH₄OH, Carbamatsalze, KHCO₃, K₂CO₃, Carbonate, Alkali- und Erdalkalibasen verwendet.

Die Beschreibung stellt weiterhin ein Verfahren zur Auftrennung des Diastereomerengemisches aus DD/LL/DL/LD-Methionylmethionin durch fraktionierte Kristallisation bereit, wobei die beiden Enantiomerenpaare DD/LL-Methionylmethionin und DL/LD-Methionylmethionin erhalten werden.

In einer bevorzugten Ausführungsform des Verfahrens der fraktionierten Kristallisation durch Ansäuern wird wie folgt vorgegangen:
a) durch Ansäuern der DD/LL/DL/LD-Methionylmethionin enthaltenden Suspension bis eine klare Lösung erhalten wird und durch schrittweises Versetzen der sauren Lösung mit einer Base bis ein Niederschlag ausfällt, wird DL/LD-Methionylmethionin als Niederschlag gewonnen,
b) aus der aus Schritt a) erhaltenen Mutterlauge wird DD/LL-Methionylmethionin gewonnen.

Dabei ist besonders bevorzugt, dass in Schritt a) das Ansäuern mit einer Säure erfolgt und ein pH-Wert von 0,1 bis 1,0, vorzugsweise ein pH-Wert von ca. 0,6 eingestellt wird, und die erhaltene klare Lösung anschließend mit einer Base auf einen pH-Wert von 5 bis 6, vorzugsweise auf einen pH-Wert von ca. 5,6 eingestellt wird. Als Säure können dabei Mineralsäuren, bevorzugt Phosphorsäure, Schwefelsäure, Salzsäure oder Kohlensäure bzw. Kohlendioxid, und/oder Carbonsäuren, insbesondere die C₁-C₄-Carbonsäuren Ameisensäure, Essigsäure, Propionsäure, Buttersäure oder Isobuttersäure, eingesetzt werden. Besonders bevorzugt wird Kohlensäure bzw. Kohlendioxid verwendet. Dabei kann die Kohlensäure bzw. das Kohlendioxid in die Reaktionsmischung unter Normaldruck oder unter Überdruck eingeleitet werden.

Zur Einstellung der basischen Bedingungen in Schritt a) wird vorzugsweise eine Base verwendet ausgewählt aus der Gruppe NH₄HCO₃, (NH₄)₂CO₃, Stickstoff haltige Basen, NH₄OH, Carbamatsalze, KHCO₃, K₂CO₃, Carbonate, Alkalibasen und Erdalkalibasen.

In einer weiteren bevorzugten Ausführungsform des Verfahrens der fraktionierten Kristallisation durch Basischstellen wird wie folgt vorgegangen:
a) durch Basischstellen der DD/LL/DL/LD-Methionylmethionin enthaltenden Suspension bis eine klare Lösung erhalten wird und durch schrittweises Versetzen der basischen Lösung mit einer Säure bis ein Niederschlag ausfällt, wird DL/LD-Methionylmethionin als Niederschlag gewönnen.
b) aus der aus Schritt a) erhaltenen Mutterlauge wird anschließend DD/LL-Methionylmethionin gewonnen.

Dabei ist besonders bevorzugt, dass in Schritt a) das Basischstellen mit einer Base erfolgt und ein pH-Wert von 7,5 bis 14, vorzugsweise ein pH-Wert von ca. 9 bis 13 eingestellt wird und die erhaltene klare Lösung anschließend mit einer Säure auf einen pH-Wert von 5 bis 6, vorzugsweise auf einen pH-Wert von ca. 5,6 eingestellt wird. Als Basen werden dabei bevorzugt Basen aus der Gruppe NH₄HCO₃, (NH₄)₂CO₃, Stickstoff haltige Basen, NH₄OH, Carbamatsalze, KHCO₃, K₂CO₃, Carbonate, Alkali- und Erdalkalibasen verwendet.

Zur Einstellung der sauren Bedingungen in Schritt a) wird vorzugsweise eine Säure verwendet aus der Gruppe der Mineralsäuren, bevorzugt die Phosphorsäure, Schwefelsäure, Salzsäure, oder Kohlensäure bzw. Kohlendioxid, und/oder aus der Gruppe der Carbonsäuren, insbesondere der C₁-C₄-Carbonsäuren Ameisensäure, Essigsäure, Propionsäure, Buttersäure und Isobuttersäure. Besonders bevorzugt wird die Kohlensäure bzw. Kohlendioxid verwendet.

In einer bevorzugten Ausführungsform des Verfahrens der fraktionierten Kristallisation erfolgt diese bei einer Temperatur von 0°C bis 100°C, vorzugsweise 5°C bis 60°C und besonders bevorzugt von 10°C bis 40°C.

Das erhaltene DD/LL-Methionylmethionin kann dabei racemisiert werden, und in das oben beschriebene Trennungsverfahren eingeführt werden, wobei die beiden Enantiomerenpaare DD/LL-Methionylmethionin und DL/LD-Methionylmethionin voneinander getrennt werden.

Alle genannten Verfahren der vorliegenden Erfindung werden bevorzugt in einem wässrigen Medium durchgeführt.

Weiterhin können die Verfahren der vorliegenden Erfindung in dem Fachmann bekannten Batch-Verfahren oder in kontinuierlichen Verfahren ausgeführt werden.

### Figuren

**Figur 1** zeigt die schematische Darstellung der enzymatischen Spaltung der Methionylmethionin-Diastereomerengemische DD/LL-**I,** DL/LD-**I** und DD/LL/DL/LD-**I**.
**Figur 2** zeigt Die schematische Darstellung der enzymatischen Spaltung der vier Methionylmethionin-Diastereomere DD-**I**, LL-**I**, DL-**I** und LD-**I** mit unterschiedlichen Spaltungsgeschwindigkeiten.
**Figur 3** zeigt die schematische Darstellung der enzymatischen Freisetzung von Methionin (D- und L-Met zusammen) aus den vier Methionylmethionin-Diastereomeren DD-**I,** LL-**I,** DL-**I** und LD-**I.**
**Figur 4** zeigt die Biotransformation von D-Methionin zu L-Methionin mit einem Enzymcocktail aus Spiegelkarpfen.
**Figur 5** zeigt das Leachingverhalten von Methionylmethionin-Diastereomerengemischen DD/LL-**I,** DL/LD-**I** und LL/DD/LD/DL-**I** im Vergleich zu Methionin, MHA und MHA-Ca.
**Figur 6** zeigt die *in vitro* Verdauung von vier verschiedenen Methionylmethionin-Diastereomeren LL-**I,** LD-**I,** DL-**I** und DD-**I** mit Verdauungsenzymen des Spiegelkarpfens.
**Figur 7** zeigt die *in vitro* Verdauung von verschiedenen Methionylmethionin-Diastereomerengemischen LL/DD-**I,** DL/LD-**I** und LL/DD/LD/DL-**I** mit Verdauungsenzymen des Spiegelkarpfens.
**Figur 8** zeigt die *in vitro* Verdauung von vier verschiedenen Methionylmethionin-Diastereomeren LL-**I**, LD-**I**, DL-**I** und DD-**I** mit Verdauungsenzymen der Regenbogenforelle.
**Figur 9** zeigt die *in vitro* Verdauung der Methionylmethionin-Diastereomerengemische LL/DD-**I**, DL/LD-**I** und LL/DD/LD/DL-**I** mit Verdauungsenzymen der Regenbogenforelle.
**Figur 10** zeigt die *in vitro* Verdauung von vier verschiedenen Methionylmethionin-Diastereomeren LL-**I,** LD-**I,** DL-**I** und DD-**I** mit Verdauungsenzymen der Whiteleg Shrimps.
**Figur 11** zeigt die *in vitro* Verdauung von verschiedenen Methionylmethionin-Diastereomerengemischen LL/DD-**I**, DL/LD-**I** und LL/DD/LD/DL-**I** mit Verdauungsenzymen der Whiteleg Shrimps.

### Beispiele

### A) Übersicht über die einzelnen Schritte und Methoden des erfindungsgemäßen Verfahrens

Das erfindungsgemäße Verfahren zur Herstellung von DL-Methionyl-DL-methionin (**I**) und die Trennung in die Diastereomerenpaare DD/LL-**I** und DL/LD-**I** werden nachfolgend eingehend beschrieben.

Das erfindungsgemäße Verfahren zur Herstellung von DL-Methionyl-DL-methionin (**I**) geht dabei aus von einer Verbindung der allgemeinen Formel **II** 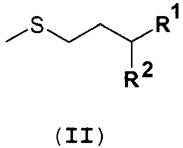 wobei
**IIa: R¹ =** COOH, **R²** = NHCONH₂
**IIb: a¹ =** CONH₂, **R²** = NHCONH₂
**IIc : R¹ =** CONH₂, **R²** = NH₂
( **IId: R¹-R² =** -CONHCONH-, nicht erfindungsgemäβ)
**IIe:** R¹ = CN, **R²** = OH
**IIf: R¹ =** CN, **R² =** NH₂
(**IIg : R¹ =** =O, **R² =** H, nicht erfindungsgemäβ
bedeutet.

Über unterschiedliche Synthesemethoden **(A, B, C, D, E, F, G, H** und **J)** wird diese Verbindung zu DL-Methionyl-DL-methionin **(I)** transformiert (siehe Schema 3). Dabei entsteht bei den Methoden **A, B, C,** und **D** das entsprechende Diketopiperazin **(III)** als Zwischenprodukt. Bei den Synthesemethoden **G, H** und **J** entsteht Methioninhydantoin als Zwischenprodukt, das direkt zu DL-Methionyl-DL-methionin **(I)** transformiert wird. Anschließend kann mit Hilfe von Methode **K** durch fraktionierte Kristallisation eine Trennung der beiden Diastereomerenpaare DD/LL-**I** und DL/LD-**I** erreicht werden (siehe Schema 3).

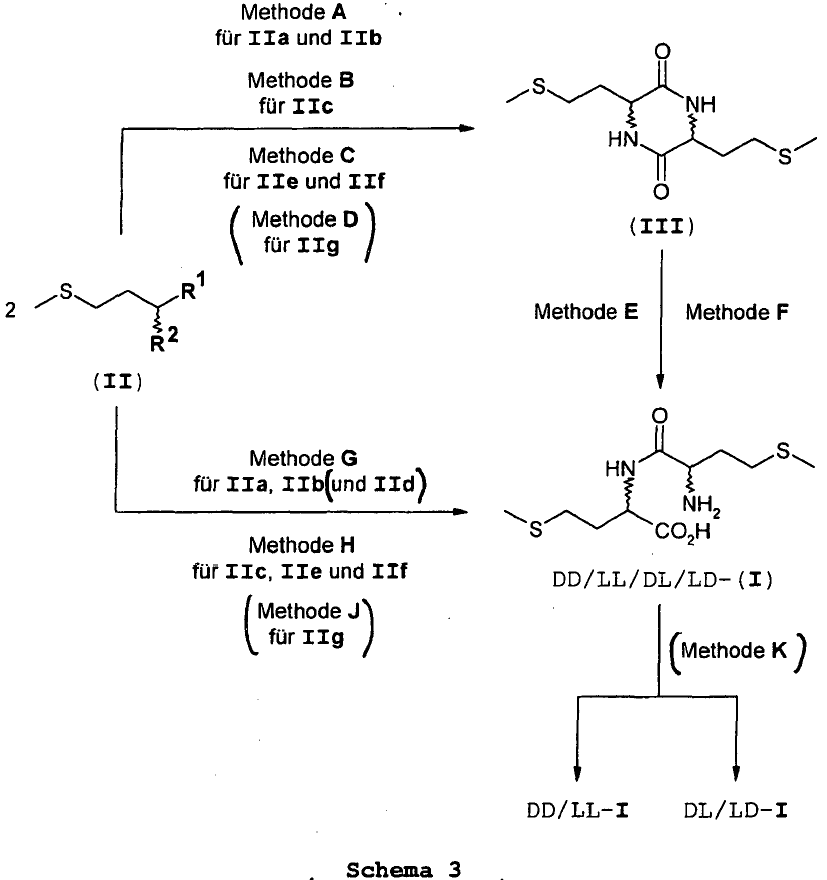

Die in Klammern gesetzten Methoden gehören nicht zur Erfindung.

### B) Synthesebeispiele:

### Beispiel 1:

### Synthese von 3,6-Bis[2-(methylthio)ethyl]-2,5-piperazindion (III) (Methionindiketopiperazin, DKP) aus N-Carbamoylmethionin (IIa) nach Methode A

17,5 g (90,0 mmol, Reinheit: 99%) N-Carbamoylmethionin **(IIa)** wurden in 150 ml Wasser gelöst und in einem 200 ml Roth-Stahlautoklav mit Magnetrührung 6 Stunden lang bei 160°C gerührt. Der Druck steigt hierbei an. Von Zeit zu Zeit wurde immer wieder so lange Gas abgelassen bis ein Druck von 7 bar erreicht wurde. Nach Beendigung der Reaktion wurde der Autoklav im Eisbad abgekühlt. Die erhaltene Suspension wurde anschließend filtriert, der abfiltrierte Feststoff mehrmals mit Wasser gewaschen und im Trockenschrank bei 50°C im Vakuum getrocknet. Die isolierte Ausbeute betrug 8,1 g (30,9 mmol) (69%) Bis[2-(methylthio)ethyl]-2,5-piperazindion (**III**), weißgelbliche Kristalle, Reinheit > 98% (HPLC), Schmelzpunkt 234-236°C.

¹H-NMR von 3,6-Bis[2-(methylthio)ethyl]-2,5-piperazindion (**III**) (500 MHz, D₆-DMSO): δ = 1,85-2,05 (m, 4H, 2 x SCH₂C**H**₂); 2,049 (s, 6H, 2 x SCH₃); 2,46-2,60 (m, 4H, 2 x SCH₂) ; 3,92-3,99 (m, 2H, 2 x CH) ; 8,213 (s, 2H, 2 x NH)

¹³C-NMR von 3,6-Bis[2-(methylthio)ethyl]-2,5-piperazindion (**III**) (125.8 MHz, D₆-DMSO): δ = 14,35 (CH₃) ; 14,38 (CH₃) ; 28,50 (CH₂S) ; 28,68 (CH₂S) ; 31,92 (**C**H₂CH₂S); 32,33 (**C**H₂CH₂S); 52,92 (CH); 52,96 (CH); 167,69 (C=O) ; 167,71 (C=O)

Elementaranalyse für C₁₀H₁₈N₂O₂S₂ (M = 262,39 g/mol) :
Berechnet: C 45,77; H 6,91; N 10,68; S 24,44
Gefunden: C 45,94; H 6,96; N 10,64; S 24,38

### Beispiel 2:

### Synthese von 3,6-Bis[2-(methylthio)ethyl]-2,5-piperazindion (III) (Methionindiketopiperazin, DKP) aus 2-[(Aminocarbonyl)amino]-4-(methylthio)butansäureamid (N-Carbamoylmethioninamid) (IIb) nach Methode A

17,4 g (90 mmol, Reinheit: 98,5%) 2-[(Aminocarbonyl)amino]-4-(methylthio)butansäureamid (**IIb**) wurden in 150 ml Wasser gelöst und in einem 200 ml Roth-Stahlautoklav mit Magnetrührung 7 Stunden lang bei 160°C gerührt. Der Druck steigt hierbei an. Von Zeit zu Zeit wurde immer wieder so lange Gas abgelassen bis ein Druck von 7 bar erreicht wurde. Nach Beendigung der Reaktion wurde der Autoklav im Eisbad abgekühlt. Die erhaltene Suspension wurde anschließend filtriert, der abfiltrierte Feststoff mehrmals mit Wasser gewaschen und im Trockenschrank bei 50°C im Vakuum getrocknet. Die isolierte Ausbeute betrug 9,2 g (35,1 mmol) (78%) Bis[2-(methylthio)ethyl]-2,5-piperazindion (**III**), weißgelbliche Kristalle, Reinheit > 98% (HPLC).

Der Schmelzpunkt und die NMR-Daten stimmten mit denen aus Beispiel 1 überein.

### Beispiel 3: (nicht erfindungsgemäβ)

### Synthese von 3,6-Bis[2-(methylthio)ethyl]-2,5-piperazindion (III) (Methionindiketopiperazin, DKP) aus 5-[2-(methylthio)ethyl]-2,9-imidazolidindion (IId) (Methioninhy-dantoin) nach Methode A und anschließendem Wiedereinsatz der Mutterlauge (Kaskadenumsetzung)

### Erster Ansatz:

Eine Suspension aus 13,4 g (0,09 mol) Methionin, 17,2 g (0,09 mol, Reinheit: 91%) Methioninhydantoin (**IId**) und 150 g Wasser wurden in einem 200 ml Roth-Stahlautoklav mit Magnetrührung vorgelegt und 6 Stunden bei 160°C gerührt, wobei der Druck auf 15 bar ansteigt. Von Zeit zu Zeit wurde der Autoklav entspannt, bis sich der Druck bei konstant 10 bar einpendelte. Danach wurde der Autoklav im Eisbad abgekühlt, die erhaltene Suspension abfiltriert und der Feststoff mit 75 ml Wasser gewaschen. Abschließend wurde der Feststoff im Vakuumtrockenschrank bei 50°C über Nacht getrocknet. Bis[2-(methylthio)ethyl]-2,5-piperazindion (**III**) wurde als weißgelbliche Kristalle isoliert.

### Nachfolgende Ansätze:

Das Waschwasser und die Mutterlauge aus dem vorherigen Ansatz wurden vereinigt und am Rotationsverdampfer bei 50°C auf 90 ml eingedickt. 17,2 g (0,09 mol, Reinheit: 91%) Methioninhydantoin (**IId**) wurde mit der aufkonzentrierten Mutterlauge aufgenommen und mit Wasser auf 150 g Lösung aufgefüllt. Die resultierende Lösung wurde in einem 200 ml Roth-Stahlautoklav mit Magnetrührung vorgelegt und 6 Stunden bei 160°C gerührt, wobei der Druck auf 15 bar ansteigt. Von Zeit zu Zeit wurde der Autoklav entspannt, bis der Druck bei konstant 10 bar blieb. Die weitere Aufarbeitung erfolgte analog dem ersten Ansatz.

### Beispiel 4:

### Synthese von 3,6-Bis[2-(methylthio)ethyl]-2,5-piperazindion (III) (Methionindiketopiperazin, DKP) aus 2-Amino-4-(methylthio)butansäureamid (Methioninamid) (IIc) nach Methode B

16,6 g (0,09 mol) 2-Amino-4-(methylthio)butansäureamid Hydrochlorid (**IIc**) und 8,7 g (0,09 mol) (NH₄)₂CO₃ wurden in 150 g Wasser gelöst und in einem 200 ml Roth-Stahlautoklav mit Magnetrührung 6 Stunden lang bei 160°C gerührt. Danach wurde der Autoklav im Eisbad abgekühlt. Die erhaltene Suspension wurde anschließend filtriert, der abfiltrierte Feststoff mehrmals mit Wasser gewaschen und im Trockenschrank bei 50°C im Vakuum getrocknet. Die isolierte Ausbeute betrug 6,5 g (24,8 mmol) (55%) Bis[2-(methylthio)ethyl]-2,5-piperazindion (**III**), weißgelbliche Kristalle, Reinheit > 98% (HPLC).

Der Schmelzpunkt und die NMR-Daten stimmten mit denen aus Beispiel 1 überein.

### Beispiel 5:

### Synthese von 3,6-Bis[2-(methylthio)ethyl]-2,5-piperazindion (III) (Methionindiketopiperazin, DKP) aus 2-Hydroxy-4-(methylthio)butannitril (3-(Methylmercapto)propionaldehyd-cyanhydrin, MMP-CH) (IIe) nach Methode C

Eine Lösung aus 30,5 g (0,232 mol) 2-Hydroxy-4-(methylthio)butannitril (**IIe**) und 360 g Wasser wurde bei RT langsam zu einer Suspension aus 22,4 g (0,283 Mol = 1,22 eq.) NH₄HCO₃ in 20 g Wasser zugetropft und 2 h lang gerührt. Das NH₄HCO₃ ging dabei in Lösung. Anschließend wurde die erhaltene Lösung 7 h lang bei 50°C und dann über Nacht bei Raumtemperatur gerührt. Das Reaktionsgemisch wurde dann in einen 500 ml-Stahlautoklav überführt, auf 160 °C aufgeheizt und 6 Stunden lang bei dieser Temperatur gerührt. Danach wurde der Autoklav im Eisbad abgekühlt, die erhaltene Suspension abfiltriert und der Feststoff mit 50 ml Wasser gewaschen. Abschließend wurde der helle Feststoff im Vakuumtrockenschrank bei 50°C über Nacht getrocknet. Die isolierte Ausbeute betrug 17,8 g (67,8 mmol) (58%) Bis[2-(methylthio)ethyl]-2,5-piperazindion (**III**), weißgelbliche Kristalle, Reinheit > 98% (HPLC).

Der Schmelzpunkt und die NMR-Daten stimmten mit denen aus Beispiel 1 überein.

### Beispiel 6:

### Synthese von 3,6-Bis[2-(methylthio)ethyl]-2,5-piperazindion (III) (Methionindiketopiperazin, DKP) aus 2-Amino-4-(methylthio)butannitril (Methioninnitril) (IIf) nach Metho-de C

In eine Lösung aus 26,2 g (0,201 mol) 2-Amino-4-(methylthio)butannitril (**IIf**) in 330 g Wasser wurde über einen Zeitraum von 3 Stunden ein mäßiger CO₂-Strom eingeleitet, wobei die Temperatur auf 45°C anstieg und sich der pH-Wert bei 8 einpendelte. Anschließend wurde über Nacht bei Raumtemperatur weitergerührt. Das Reaktionsgemisch wurde am nächsten Morgen in einen 500 ml-Stahlautoklav überführt, auf 160°C aufgeheizt und 6 Stunden bei dieser Temperatur gerührt. Danach wurde der Autoklav im Eisbad abgekühlt, die erhaltene Suspension abfiltriert, der Feststoff mit 50 ml Wasser gewaschen und im Vakuumtrockenschrank bei 50°C über Nacht getrocknet. Die isolierte Ausbeute betrug 15,7 g (59,7 mmol) (59%) Bis[2-(methylthio)ethyl]-2,5-piperazindion (**III**), weißgelbliche Kristalle, Reinheit > 98% (HPLC).

Der Schmelzpunkt und die NMR-Daten stimmten mit denen aus Beispiel 1 überein.

### Beispiel 7: (nicht erfindungsgemäβ)

### Synthese von 3,6-Bis[2-(methylthio)ethyl]-2,5-piperazindion (III) (Methionindiketopiperazin, DKP) aus 3-(methylthio)propanaldehyd (3-(Methylmercapto)propionaldehyd, MMP) (IIg) nach Methode D

66,0 g (0,68 mol) (NH₄)₂CO₃ wurden in 100 g Wasser vorgelegt und im Eisbad auf 5°C abgekühlt. Anschließend wurden innerhalb von 25 Minuten 16,6 g (0,61 mol) frisch destillierte Blausäure zugetropft, wobei die Temperatur der Suspension im Bereich von 5 bis 10°C gehalten wurde. Nach Zugabe von 860 g Wasser wurden bei 10°C 60,3 g (0,58 Mol) 3-(Methylthio)propionaldehyd (**IIg**) über einen Zeitraum von 80 Min. zugetropft. Der pH-Wert blieb dabei konstant im Bereich von 8,5 bis 9. Anschließend wurde das Reaktionsgemisch auf 50°C aufgewärmt und 7 Stunden lang bei dieser Temperatur gerührt. Nach Beendigung der Reaktion wurde das Reaktionsgemisch im Eisbad auf 5°C abgekühlt und über Nacht im Kühlschrank gelagert. Am nächsten Morgen wurde das Gemisch in einen 2 1-Stahlautoklav überführt, auf 160°C aufgeheizt und 6 Stunden bei dieser Temperatur gerührt. Danach wurde der Autoklav im Eisbad abgekühlt, die erhaltene Suspension abfiltriert, mit 150 ml Wasser gewaschen und der Feststoff im Vakuumtrockenschrank bei 50°C über Nacht getrocknet. Die isolierte Ausbeute betrug 48,6 g (185,2 mmol) (64%) Bis[2-(methylthio)ethyl]-2,5-piperazindion (**III**), weißgelbliche Kristalle, Reinheit > 98% (HPLC).

Der Schmelzpunkt und die NMR-Daten stimmten mit denen aus Beispiel 1 überein.

### Beispiel 8:

### Synthese von DD/LL/DL/LD-Methionylmethionin (I) aus 3,6-Bis[2-(methylthio)ethyl]-2,5-piperazindion (III) (Methionindiketopiperazin, DKP) mit konzentrierter Salzsäure nach Methode E

655,9 g (2,50 mol) 3,6-Bis[2-(methylthio)ethyl]-2,5-piperazindion (**III**) (DKP) wurden mit 1661 g Wasser suspendiert. Unter Rühren wurden 271,0 g konz. Salzsäure ganz langsam zugetropft und anschließend vorsichtig unter sehr starkem Rühren bis zum Rückfluss erhitzt. Dabei kann es zu starker Schaumbildung kommen. Die Reaktionsmischung wurde 5,5 Stunden lang am Rückfluss erhitzt, wodurch der gesamte Feststoff in Lösung ging. Während dem anschließenden Abkühlen fiel nichtumgesetztes DKP (**III**) aus, welches abfiltriert wurde. Dieses DKP kann für weitere Verseifungen in späteren Umsetzungen wieder eingesetzt werden. Das Filtrat wurde anschließend in einem Becherglas mit Eisbad mit 32%igem Ammoniakwasser auf pH 6 eingestellt. Dabei fällt ein DD/LL/DL/LD-Methionylmethionin (**I**) als 50:50-Gemisch der beiden Diastereomerenpaare (DL/LD-Met-Met) (DL/DL-**I**) bzw. (DD/LL-Met-Met) (DD/LL-**I**) als dicker Kristallbrei aus. Abschließend wurde im Trockenschrank bei 60°C im Vakuum getrocknet. Ausbeute: 601,0 g (2,14 mol) (85,7%) DD/LL/DL/LD-Methionylmethionin (**I**), leicht gelblicher Feststoff, Reinheit 98% (HPLC).

¹H-NMR von DD/LL/DL/LD-Methionylmethionin (**I**) (500 MHz, D₆-DMSO+HCl) : δ = 1,86-2,16 (m, 4H, 2 x SCH₂C**H**₂) ; 2,050 (s, 3H, SCH₃); 2,060 (s, 3H, SCH₃); 2,44-2,64 (m, 4H, 2 x SCH₂); 2,90-4,00 (m, 1H, CH); 4,32-4,42 (m, 1H, CH); 8,45 (bs, 3H, NH₃⁺) ; 8,98-9,08 (m, 1H, 2 x NH)

¹³C-NMR von DD/LL/DL/LD-Methionylmethionin (**I**) (125.8 MHz, D₆-DMSO+HCl): δ = 14,33 (CH₃) ; 14,38 (CH₃); 27,74; 27,94; 29,51; 30,04; 30,13; 30,89; 30,95; 51,00; 51,29; 51,54 (CH, CH₂); 168,05 (CONH); 168,19 (CONH); 172,55 (COOH); 172,62 (COOH)

Elementaranalyse für C₁₀H₂₀N₂O₃S₂ (M = 280,41 g/mol):
Berechnet: C 42,83; H 7,19; N 9,99; S 22,87
Gefunden: C 42,61; H 7,19; N 10,06; S 22,72

### Beispiel 9:

### Technische Synthese von DD/LL/DL/LD-Methionylmethionin (I) aus 3,6-Bis[2-(methylthio)ethyl]-2,5-piperazindion (III) (Methionindiketopiperazin, DKP) mit konzentrierter Salzsäure nach Methode E

In einem 500 l emaillierten Rührwerksbehälter wurden 500 1 Wasser vorgelegt, 32 1 konzentrierte Salzsäure sowie 78,6 kg 3,6-Bis[2-(methylthio)ethyl]-2,5-piperazindion (III) (DKP) zugegeben und der Apparat dicht verschlossen. Dann wurde 2 Stunden lang unter Rühren auf 110 °C erhitzt, wobei der Druck auf 2,5 bar anstieg und das DKP (**III**) praktisch vollständig in Lösung ging. Nach beendeter Reaktion wurde auf 20°C abgekühlt und das nicht umgesetzte DKP auf einer Zentrifuge abgeschleudert. Der Feststoff wurde mit 10 l Wasser nachgewaschen. Das Filtrat und Waschwasser wurden dann in einem 800 l Container gesammelt und anschließend wieder in einen 500 l Rührwerksbehälter eingezogen. Nach Zugabe von 2 kg Aktivkohle wurde bei 20°C 30 Min. lang gerührt. Die Suspension wurde anschließend über eine Filterpresse in einen weiteren 500 l Rührwerksbehälter filtriert. Durch Zugabe von ca. 28 1 konzentrierter Ammoniaklösung wurde bei pH 6 anschließend das DD/LL/DL/LD-Methionylmethionin (**I**) ausgefällt. Dabei fällt zu Beginn bevorzugt das schwerlöslichere racemische Diastereomerenpaar DL/LD-Methionylmethionin (DL/LD-**I**) aus. Dieses wurde abgeschleudert und die Mutterlauge zusammen mit Waschwasser im Dampfstrahlvakuum bei maximal 40°C Innentemperatur auf ein Viertel des ursprünglichen Volumens eingeengt. Dabei kristallisierte das leichtlöslichere racemische Diastereomerenpaar DD/LL-Methionylmethionin (DD/LL-**I**)zusammen mit geringen Mengen des schwerlöslichen DL/LD-**I** aus. Nach beendeter Destillation wurde auf 20°C abgekühlt und geschleudert. Die abgetrennte Mutterlauge und Waschwasser wurden verworfen. Beide Fraktionen werden bei 70°C im Vakuum getrocknet. Ingesamt konnten 64,2 kg (78%) DD/LL/DL/LD-Methionylmethionin (**I**) als Diastereomerengemisch erhalten werden. Reinheit > 98% (HPLC).

Der Schmelzpunkt und die NMR-Daten stimmten mit denen aus Beispiel 8 überein.

### Beispiel 10:

### Synthese von DD/LL/DL/LD-Methionylmethionin (I) aus 3,6-Bis[2-(methylthio)ethyl]-2,5-piperazindion (III) (Methionindiketopiperazin, DKP) unter alkalischen Bedingungen z.B. mit Ammoniak nach Methode F

65,6 g (0,25 Mol) 3,6-Bis[2-(methylthio)ethyl]-2,5-piperazindion (**III**) (DKP), 70 ml 25%ige Ammoniaklösung und 500 ml Wasser werden 2 Stunden in einem Autoklaven auf 150°C erhitzt. Nach Abkühlen wird das nicht umgesetzte DKP (**III**) (16,0 g = 24,4%) abgesaugt. Dieses kann in einem nächsten Ansatz wieder eingesetzt werden. Das Filtrat wurde im Rotationsverdampfer bei einer Wassertemperatur von 80-90°C soweit eingeengt, bis die ersten Kristalle ausfielen. Nach Abkühlen und Stehenlassen über Nacht konnten nach Filtration und Trocknung ingesamt 49,3 g (70,3%) DD/LL/DL/LD-Methionylmethionin (**I**) als 50:50-Gemisch der beiden Diastereomerenpaare DL/DL-**I** bzw. DD/LL-**I** als weißer Feststoff isoliert werden. Reinheit 98% (HPLC).

Der Schmelzpunkt und die NMR-Daten stimmten mit denen aus Beispiel 8 überein.

### Beispiel 11: (nicht erfindungsgemäβ)

### Aufreinigung von DD/LL/DL/LD-Methionylmethionin (I)

500 g DD/LL/DL/LD-Methionylmethionin (**I**) wurden in 7800 g VE-Wasser suspendiert (pH 5,3). Bei 26°C wurde mit 346,6 g 50 Gew.-% Schwefelsäure auf pH 1,0 eingestellt. Das Methionylmethionin löste sich dabei vollständig auf. Zur Klärung wurden 18 g Aktivkohle zur gelblichen, trüben Lösung gegeben und 60 Minuten lang gerührt. Die Aktivkohle wurde abfiltriert und die wasserklare, farblose Lösung mit 228 g 32 Gew.-% Ammoniaklösung auf pH 5,6 eingestellt. Die Lösung wurde über Nacht stehen gelassen. Der ausgefallene weiße Feststoff wurde abgesaugt und im Trockenschrank bei 50°C im Vakuum getrocknet. Ausbeute: 460,5 g (92%) DD/LL/DL/LD-Methionylmethionin (**I**), strahlend weißer Feststoff, Reinheit > 99% (HPLC).

Die NMR-Daten stimmten mit denen aus Beispiel 8 überein.

### Beispiel 12:

### Synthese von DD/LL/DL/LD-Methionylmethionin (I) aus N-Carbamoylmethionin (IIa) und DL-Methionin mit KOH nach Methode G

13,4 g (0,09 mol) DL-Methionin, 17,5 g (0,09 mol, Reinheit: 99%) N-Carbamoylmethionin (**IIa**) und 11,9 g (0,18 mol) 85%iges KOH wurden in 150 ml Wasser gelöst und in einem 200 ml Roth-Stahlautoklav mit Magnetrührung 5 Stunden bei 150°C gerührt, wobei der Druck auf 6 bar anstieg. Nach Beendigung der Reaktion wurde der Autoklav abgekühlt, das ausgefallene 3,6-Bis[2-(methylthio)ethyl]-2,5-piperazindion (**III**) (Methionindiketopiperazin, DKP) abfiltriert und mit etwas Wasser gewaschen. Das Waschwasser und die Mutterlauge wurden vereinigt und am Rotationsverdampfer bei 40°C auf ein Volumen von 130 ml eingeengt. In die erhaltene Lösung wurde anschließend ein mäßiger CO₂-Strom eingeleitet, bis ein pH-Wert von 6,4 erreicht wurde und ein weißer Feststoff ausfiel. Dieser wurde abfiltriert, mit wenig kaltem Wasser gewaschen und im Vakuumtrockenschrank bei 50°C über Nacht getrocknet. Die isolierte Ausbeute betrug 11,4 g (40,6 mmol) (45%) DD/LL/DL/LD-Methionylmethionin (**I**), weißer Feststoff, Reinheit > 98% (HPLC).

Die NMR-Daten stimmten mit denen aus Beispiel 8 überein.

### Beispiel 13: (nicht erfindungsgemäβ)

### Synthese von DD/LL/DL/LD-Methionylmethionin (I) aus 5-[2-(methylthio)ethyl]-2,4-imidazolidindion (IId) (Methioninhydantoin) und DL-Methionin mit KOH nach Methode G

13,4 g (0,09 mol) DL-Methionin, 17,2 g (0,09 mol, Reinheit: 91%) Methioninhydantoin (**IId**) und 8,9 g (0,135 mol) 85%iges KOH wurden in 150 ml Wasser gelöst und in einem 200 ml Roth-Stahlautoklav mit Magnetrührung 5 Stunden bei 150°C gerührt, wobei der Druck auf 8 bar ansteigt. Nach Beendigung der Reaktion wurde der Autoklav abgekühlt, die erhaltene Suspension abfiltriert und das ausgefallene 3,6-Bis[2-(methylthio)ethyl]-2,5-piperazindion (**III**) (Methionindiketopiperazin, DKP) mehrmals mit wenig Wasser gewaschen. Mutterlauge und Waschwasser wurden vereinigt und die resultierende Lösung am Rotationsverdampfer bei 40°C auf ein Volumen von 125 ml eingeengt. Das Konzentrat wurde mit konzentrierter Salzsäure vorsichtig neutralisiert. Bei einem pH-Wert von 5,8 fiel während des Rührens über Nacht bei Raumtemperatur ein weißer Feststoff aus. Dieser wurde abfiltriert, mit wenig kaltem Wasser gewaschen und im Vakuumtrockenschrank bei 50°C über Nacht getrocknet. Die isolierte Ausbeute betrug 17,5 g (62,4 mmol) (69%) DD/LL/DL/LD-Methionylmethionin (I), weißer Feststoff, Reinheit > 98% (HPLC).

Die NMR-Daten stimmten mit denen aus Beispiel 8 überein.

### Beispiel 14: (nicht erfindungsgemäβ)

### Synthese von DD/LL/DL/LD-Methionylmethionin (I) aus 5-[2-(methylthio)ethyl]-2,4-imidazolidindion (IId) (Methioninhydantoin) und DL-Methionin mit K₂CO₃ nach Methode G

13,4 g (0,09 mol) DL-Methionin, 17,2 g (0,09 mol, Reinheit: 91%) Methioninhydantoin (**IId**) und 12,4 g (0,09 mol) K₂CO₃ wurden in 150 ml Wasser gelöst und in einem 200 ml Roth-Stahlautoklav mit Magnetrührung 5 Stunden bei 150°C gerührt, wobei der Druck auf 12 bar ansteigt. Nach Beendigung der Reaktion wurde der Autoklav abgekühlt, das ausgefallene 3,6-Bis[2-(methylthio)ethyl]-2,5-piperazindion (**III**) (Methionindiketopiperazin, DKP) abfiltriert und mit etwas Wasser gewaschen. Das Waschwasser und die Mutterlauge wurden vereinigt und am Rotationsverdampfer bei 40°C auf ein Volumen von 135 ml eingeengt. In die erhaltene Lösung wurde anschließend ein mäßiger CO₂-Strom eingeleitet, bis ein pH-Wert von 6,8 erreicht wurde und ein weißer Feststoff ausfiel. Dieser wurde abfiltriert, mit wenig kaltem Wasser gewaschen und im Vakuumtrockenschrank bei 50°C über Nacht getrocknet. Ausbeute: 14,3 g (60,0 mmol) (57%) DD/LL/DL/LD-Methionylmethionin (I), weißer Feststoff, Reinheit > 99% (HPLC).

Die NMR-Daten stimmten mit denen aus Beispiel 8 überein.

### Beispiel 15: (nicht erfindungsgemäβ)

### Synthese von DD/LL/DL/LD-Methionylmethionin (I) aus 5-[2-(methylthio)ethyl]-2,4-imidazolidindion (IId) (Methioninhydantoin) und DL-Methionin mit KHCO₃ nach Methode G

13,4 g (0,09 mol) DL-Methionin, 17,2 g (0,09 mol, Reinheit: 91%) Methioninhydantoin (**IId**) und 9,1 g (0,09 mol) KHCO₃ wurden in 150 ml Wasser gelöst und in einem 200 ml Roth-Stahlautoklav mit Magnetrührung 5 Stunden bei 150°C gerührt, wobei der Druck auf 12 bar ansteigt. Nach Beendigung der Reaktion wurde der Autoklav abgekühlt, das ausgefallene 3,6-Bis[2-(methylthio)ethyl]-2,5-piperazindion (**III**) (Methionindiketopiperazin, DKP) abfiltriert und mit etwas Wasser gewaschen. Das Waschwasser und die Mutterlauge wurden vereinigt und am Rotationsverdampfer bei 40°C auf ein Volumen von 120 ml eingeengt. In die erhaltene Lösung wurde anschließend ein mäßiger CO₂-Strom eingeleitet, bis ein pH-Wert von 6,3 erreicht wurde und ein weißer Feststoff ausfiel. Dieser wurde abfiltriert, mit wenig kaltem Wasser gewaschen und im Vakuumtrockenschrank bei 50°C über Nacht getrocknet. Ausbeute: 16,0 g (57,1 mmol) (63%) DD/LL/DL/LD-Methionylmethionin (**I**), weißer Feststoff, Reinheit > 99% (HPLC) .

Die NMR-Daten stimmten mit denen aus Beispiel 8 überein.

### Beispiel 16:

### Synthese von DD/LL/DL/LD-Methionylmethionin (I) aus 2-Amino-4-(methylthio)butansäureamid (IIc) (Methioninamid) und DL-Methionin mit (NH₄)₂CO₃ nach Methode H

8,3 g (0,045 mol) 2-Amino-4-(methylthio)butansäureamid (**IIc**) Hydrochlorid, 6,7 g (0,045 mol) Methionin, 4,3 g (0,045 mol) (NH₄) ₂CO₃ und 3,0 g (0,045 mol) 85%iges KOH wurden in 75 g Wasser gelöst und in einem 200 ml Roth-Stahlautoklav mit Magnetrührung 6 Stunden bei 160°C gerührt. Danach wurde der Autoklav im Eisbad abgekühlt, die erhaltene Suspension abfiltriert und das ausgefallene 3,6-Bis[2-(methylthio)ethyl]-2,5-piperazindion (**III**) (Methionindiketopiperazin, DKP) mit wenig Wasser gewaschen. Das Waschwasser und die Mutterlauge wurden vereinigt, am Rotationsverdampfer bei 40°C auf ein Volumen von 70 ml eingeengt. In die erhaltene Lösung wurde anschließend ein mäßiger CO₂-Strom eingeleitet, bis ein pH-Wert von 6,3 erreicht wurde und ein weißer Feststoff ausfiel. Dieser wurde abfiltriert, mit wenig kaltem Wasser gewaschen und im Vakuumtrockenschrank bei 50°C über Nacht getrocknet. Ausbeute: 7,8 g (27,8 mmol) (62%) DD/LL/DL/LD-Methionylmethionin (**I**), weißer Feststoff, Reinheit > 98% (HPLC).

Die NMR-Daten stimmten mit denen aus Beispiel 8 überein.

### Beispiel 17:

### Synthese von DD/LL/DL/LD-Methionylmethionin (I) aus 2-Hydroxy-4-(methylthio)butannitril (IIe) (3-(Methylmercapto)propionaldehydcyanhydrin, MMP-CH) und DL-Methionin mit NH₄HCO₃ nach Methode H

15,2 g (0,116 mol) 2-Hydroxy-4-(methylthio)butannitril (**IIe**) wurden bei RT langsam zu einer Suspension aus 11,1 g (0,141 Mol = 1,22 eq.) NH₄HCO₃ in 10 g Wasser zugetropft und 2 h lang gerührt. Das NH₄HCO₃ ging dabei in Lösung. Anschließend wurden 180 g Wasser zugegeben und die erhaltene Lösung 7 h bei 50°C und über Nacht bei Raumtemperatur gerührt. Am nächsten Morgen wurden 17,3 g (0,116 mol) Methionin, 7,7 g (0,116 mol) 85%ges KOH sowie weitere 180 g Wasser zugegeben und das Reaktionsgemisch in einen 1 1-Stahlautoklav überführt, auf 160°C aufgeheizt und 6 Stunden lang bei dieser Temperatur gerührt. Danach wurde der Autoklav im Eisbad abgekühlt, die erhaltene Suspension abfiltriert und das ausgefallene 3,6-Bis[2-(methylthio)ethyl]-2,5-piperazindion (**III**) (Methionindiketopiperazin, DKP) mit 100 ml Wasser gewaschen. Mutterlauge und Waschwasser wurden vereinigt und die resultierende Lösung am Rotationsverdampfer bei 40°C auf ein Volumen von 160 ml eingeengt. Das Konzentrat wurde mit 50%iger Schwefelsäure vorsichtig neutralisiert. Bei einem pH-Wert von 5,4 fiel während des Rührens über Nacht bei Raumtemperatur ein weißer Feststoff aus. Dieser wurde abfiltriert, mit wenig kaltem Wasser gewaschen und im Vakuumtrockenschrank bei 50°C über Nacht getrocknet. Ausbeute: 15,2 g (54,2 mmol) (47%) DD/LL/DL/LD-Methionylmethionin (**I**), weißer Feststoff, Reinheit > 99% (HPLC).

Die NMR-Daten stimmten mit denen aus Beispiel 8 überein.

### Beispiel 18:

### Synthese von DD/LL/DL/LD-Methionylmethionin (I) aus 2-Amino-4-(methylthio)butannitril (IIf) (Methioninnitril) mit CO₂ und DL-Methionin nach Methode H

In eine Lösung von 26,2 g (0,201 mol) 2-Amino-4-(methylthio)butannitril (**IIf**) in 330 g Wasser wurde über einen Zeitraum von 3 Stunden ein mäßiger CO₂-Strom eingeleitet, wobei die Temperatur auf 45°C anstieg und der pH-Wert sich bei 8 einpendelte. Anschließend wurde über Nacht bei Raumtemperatur weitergerührt. Das Reaktionsgemisch wurde am nächsten Morgen mit 30,0 g (0,201 mol) Methionin und 13,3 g (0,201 mol) 85%iges KOH versetzt und in einen 1 1-Stahlautoklav überführt, auf 160°C aufgeheizt und 6 Stunden bei dieser Temperatur gerührt. Danach wurde der Autoklav im Eisbad abgekühlt, die erhaltene Suspension abfiltriert und das ausgefallene 3,6-Bis[2-(methylthio)ethyl]-2,5-piperazindion (**III**) (Methionindiketopiperazin, DKP) mit wenig Wasser gewaschen. Das Waschwasser und die Mutterlauge wurden vereinigt, am Rotationsverdampfer bei 40°C auf ein Volumen von 280 ml eingeengt. In die erhaltene Lösung wurde anschließend ein mäßiger CO₂-Strom eingeleitet, bis ein pH-Wert von 6,0 erreicht wurde und ein weißer Feststoff ausfiel. Dieser wurde abfiltriert, mit wenig kaltem Wasser gewaschen und im Vakuumtrockenschrank bei 50°C über Nacht getrocknet. Ausbeute: 32,7 g (116,6 mmol) (58%) DD/LL/DL/LD-Methionylmethionin (I), weißer Feststoff, Reinheit > 98% (HPLC).

Die NMR-Daten stimmten mit denen aus Beispiel 8 überein.

### Beispiel 19: (nicht erfindungsgemäβ)

### Synthese von DD/LL/DL/LD-Methionylmethionin (I) aus 3-(Methylthio)propanaldehyd (IIg) (MMP) mit Blausäure, Ammoniumcarbonat und DL-Methionin nach Methode J

66,0 g (0,68 mol) (NH₄) ₂CO₃ wurden in 100 g Wasser vorgelegt und im Eisbad auf 5°C abgekühlt. Anschließend wurden innerhalb 25 Min. 16,55 g (0,612 Mol) frisch destillierte Blausäure zugetropft, wobei die Temperatur der Suspension auf 5 bis 10°C gehalten wurde. Nach Zugabe von 500 g Wasser wurden 60,3 g (0,58 Mol) 3-(Methylthio)propionaldehyd (**IIg**) über einen Zeitraum von 80 Min. bei 10°C zugetropft. Der pH-Wert blieb dabei konstant im Bereich von 8,5 bis 9. Anschließend wurde das Reaktionsgemisch auf 50°C aufgewärmt und 7 Stunden lang bei dieser Temperatur gerührt. Nach Beendigung der Reaktion wurde das Reaktionsgemisch im Eisbad auf 5°C abgekühlt und über Nach im Kühlschrank gelagert. Am nächsten Morgen wurden 86,5 g (0,58 mol) 2-Amino-4-(methylthio)butansäure (Methionin), 38,3 g (0,58 mol) 85%iges KOH (0,58 mol), sowie weitere 530 g Wasser zugegeben. Das Gemisch wurde in einen 2 1-Stahlautoklav überführt, auf 160°C aufgeheizt und 6 Stunden bei dieser Temperatur gerührt. Danach wurde der Autoklav im Eisbad abgekühlt, die erhaltene Suspension abfiltriert und das ausgefallene 3,6-Bis[2-(methylthio)ethyl]-2,5-piperazindion (**I-II**) (Methionindiketopiperazin, DKP) mit wenig Wasser gewaschen. Das Waschwasser und die Mutterlauge wurden vereinigt, am Rotationsverdampfer bei 40°C auf ein Volumen von 800 ml eingeengt. In die erhaltene Lösung wurde anschließend ein mäßiger CO₂-Strom eingeleitet, bis ein pH-Wert von 6,0 erreicht wurde und ein weißer Feststoff ausfiel. Dieser wurde abfiltriert, mit wenig kaltem Wasser gewaschen und im Vakuumtrockenschrank bei 50°C über Nacht getrocknet. Ausbeute: 85,1 g (0,30 mol) (52°) DD/LL/DL/LD-Methionylmethionin (**I**), weißer Feststoff, Reinheit > 98% (HPLC) .

Die NMR-Daten stimmten mit denen aus Beispiel 8 überein

Beispiel 20: (nicht erfindungsgemäβ)

### Trennung der beiden Diastereomerenpaare DD/LL-Methionylmethionin (DD/LL-I)und DL/LD-Methionylmethionin (DL/LD-I) durch fraktionierte Kristallisation aus DD/LL/DL/LD-Methionylmethionin (I) nach Methode K

### a) DL/LD-Methionylmethionin (DL/LD-I):

290,4 g DD/LL/DL/LD-Methionylmethionin (**I**) (50:50 Mischung aus DD/LL-**I** und DL/LD-**I**) wurden in 2614 g VE-Wasser suspendiert und mit 381,7 g 50 Gew.-% Schwefelsäure auf pH 0,6 eingestellt. Die klare, farblose Lösung wurde mit 265,9 g 32 Gew.-% Ammoniak-lösung auf pH 5,6 eingestellt und der entstandene weiße Niederschlag abgesaugt (580,9 g feucht). Abschließend wurde der Feststoff im Trockenschrank bei 50°C im Vakuum getrocknet. Die Ausbeute betrug 126,2 g (86,9%) DL/LD-Methionylmethionin (DL/LD-**I**), weißer Feststoff, Reinheit > 98% (HPLC), Schmelzbereich 232-233°C (Zers.).

¹H-NMR von DL/LD-Methionylmethionin (DL/LD-**I**) (500 MHz, D₆-DMSO+HCl) : 1,88-2,12 (m, 4H, 2 x SCH₂C**H**₂) ; 2, 031 (s, 3H, CH₃) ; 2,041 (s, 3H, CH₃) ; 2, 48-2, 56 (m, 4H, 2 x SCH₂) ; 3,87-3,95 (m, 1H, CH) ; 4,30-4,38 (m, 1H, CH); 8,429 (d, 3H, ³*J* = 4,4Hz, NH₃⁺) ; 9,034 (d, 1H, ³J = 8,0Hz, NH)
¹³C-NMR von DL/LD-Methionylmethionin (DL/LD-**I**) (125.8 MHz, D₆-DMSO+HCl) : 14,57 (CH₃) ; 14,62 (CH₃) ; 28, 19: 29,75; 30,28; 31,19; 51,25 (CH); 51,79 (CH); 168,29 (CONH); 172,80 (COOH) Löslichkeit(Wasser, 20'C): 0,4 g/l

### b) DD/LL-Methionylmethionin (DD/LL-I):

Die farblose Mutterlauge aus a) wurde am Rotationsverdampfer bei 35°C und Wasserpumpenvakuum aufkonzentriert. Man erhielt eine weiße Suspension. Der weiße Feststoff aus Ammoniumsulfat, Resten an DL/LD-**I** und Zielverbindung wurde anschließend abgesaugt und im Vakuum bei 50°C getrocknet. Zur Trennung der drei Feststoffe wurde das Gemisch in VE-Wasser suspendiert und ausgerührt. Das ungelöste DL/LD-**I** wurde abgesaugt und die Mutterlauge am Rotationsverdampfer bei 50°C und Wasserpumpenvakuum auf ca. ein Fünftel aufkonzentriert. Nach längerem Stehen kristallisierte DD/LL-Methionylmethionin (DD/LL-**I**) als weißer Feststoff aus. Abschließend wurde abgesaugt und im Vakuumtrockenschrank bei 50°C getrocknet. Die Ausbeute betrug 78,2 g (53,9%) bezogen auf DD/LL-Methionylmethionin (DD/LL-**I**), weißer Feststoff, > 96% (HPLC), Schmelzbereich 226-227°C (Zersetzung).

¹H-NMR von DD/LL-Methionylmethionin (DD/LL-**I**) (500 MHz, D₆-DMSO+HCl) : 1,84-2,12 (m, 4H, 2 x SCH₂C**H**₂) ; 2, 044 (s, 3H, CH₃) ; 2,046 (s, 3H, CH₃) ; 2,48-2,62 (m, 4H, 2 x SCH₂) ; 3,89-3,97 (m, 1H, CH); 4,33-4,40 (m, 1H, CH); 8,422 (d, 3H, ³*J* = 4,0Hz, NH₃⁺) ; 9,065 (d, 1H, ³J = 7,5Hz, NH)

¹³C-NMR von DD/LL-Methionylmethionin (DD/LL-**I**) (125.8 MHz, D₆-DMSO+HCl) : 14,56 (CH₃) ; 14,57 (CH₃) ; 27,97; 29,73; 30,35; 31,11; 51,22 (CH); 51,50 (CH); 168,41 (CONH); 172,83 (COOH)

Löslichkeit(Wasser, 20°C): 21,0 g/l

### Beispiel 21: (nicht erfindungsgemäβ)

### Racemisierung der beiden Diastereomerenpaare DD/LL-Methionylmethionin (DD/LL-I)und DL/LD-Methionylmethionin (DL/LD-I) unter basischen Bedingungen

### a) Racemisierung von DL/LD-Methionylmethionin (DL/LD-I)

12,6 g (45,0 mmol) des Diastereomerenpaars DL/LD-Methionylmethionin (DL/LD-**I**) wurden zusammen mit 3,1 g (22,5 mmol) K₂CO₃ in einem 200 ml Roth-Laborreaktor in 75 ml Wasser gelöst und unter Rühren auf 160°C erhitzt. Der Druck stieg dabei auf 7 bar an. Nach 6 Stunden bei dieser Temperatur wurde der Autoklav im Eisbad abgekühlt. Die erhaltene Suspension wurde anschließend filtriert, der abfiltrierte Feststoff mehrmals mit Wasser gewaschen und im Trockenschrank bei 50°C im Vakuum getrocknet. Die isolierte Ausbeute betrug 6,5 g (24, 8 mmol) (55%) Bis [2-(methylthio)ethyl]-2,5-piperazindion (**III**), weißgelbliche Kristalle, Reinheit > 98%, Schmelzpunkt 234-236°C; Diastereomerenverhältnis: 52:48 (DD/LL-**III** : *meso*-**III**). Das Waschwasser und die Mutterlauge wurden vereinigt und am Rotationsverdampfer bei 40°C auf ein Volumen von 25 ml eingeengt. In die erhaltene Lösung wurde anschließend ein mäßiger CO₂-Strom eingeleitet, bis der pH-Wert bei 6,0 erreicht wurde und ein weißer Feststoff ausfiel. Dieser wurde abfiltriert, mit wenig kaltem Wasser gewaschen und im Vakuumtrockenschrank bei 50°C über Nacht getrocknet. Die isolierte Ausbeute betrug 5,7 g (20,3 mmol) (45%) DD/LL/DL/LD-Methionylmethionin (**I**), weißer Feststoff, Reinheit > 98% (HPLC) .

Die NMR-Daten stimmten mit denen aus Beispiel 8 überein

### a) Racemisierung von DL/LD-Methionylmethionin (DD/LL-I)

12,6 g (45,0 mmol) DD/LL-Methionylmethionin (DD/LL-**I**) wurden zusammen mit 4,5 g (45,0 mmol) KHCO₃ in einem 200 ml Roth-Laborreaktor.in 75 ml Wasser gelöst und unter Rühren auf 160°C erhitzt. Der Druck stieg auf 7 bar an, nach 6 Stunden bei dieser Temperatur wurde der Autoklav im Eisbad abgekühlt. Die erhaltene Suspension wurde anschließend filtriert, der abfiltrierte Feststoff mehrmals mit Wasser gewaschen und im Trockenschrank bei 50°C im Vakuum getrocknet. Die isolierte Ausbeute betrug 6,0 g (22,9 mmol) (51%) Bis[2-(methylthio)ethyl]-2,5-piperazindion (**III**), weißgelbliche Kristalle, Reinheit > 98% (HPLC), Schmelzpunkt 233-236°C; Diastereomerenverhältnis: 54:46 (DD/LL-**III** : *meso-***III**). Das Waschwasser und die Mutterlauge wurden vereinigt und am Rotationsverdampfer bei 40°C auf ein Volumen von 25 ml eingeengt. In die erhaltene Lösung wurde anschließend ein mäßiger CO₂-Strom eingeleitet, bis ein pH-Wert von 6,0 erreicht wurde und ein weißer Feststoff ausfiel. Dieser wurde abfiltriert, mit wenig kaltem Wasser gewaschen und im Vakuumtrockenschrank bei 50°C über Nacht getrocknet. Die isolierte Ausbeute betrug 5,5 g (19,6 mmol) (44%) DD/LL/DL/LD-Methionylmethionin (**I**), weißer Feststoff, Reinheit > 98% (HPLC).

Die NMR-Daten stimmten mit denen aus Beispiel 8 überein.

### Beispiel 22:

### In vitro Verdauungsversuche von DL-Methionyl-DL-methionin (I) mit Verdauungsenzymen aus omnivoren Karpfen

### a) Isolierung der Verdauungsenzyme aus Spiegelkarpfen (Cyprinus carpio morpha noblis)

Die Isolierung der Verdauungsenzyme wurde in Anlehnung an die Methode von EID und MATTY (Aquaculture 1989, 79, 111-119) durchgeführt. Dazu wurde der Darm von fünf einjährigen Spiegelkarpfen (*Cyprinus carpio morpha noblis*) freigelegt, mit Wasser gespült, längs aufgeschnitten und jeweils die Darmschleimhaut abgekratzt. Diese wurde zusammen mit zerstoßenem Eis mit einem Mixgerät zerkleinert. Die resultierende Suspension wurde mit einem Ultraschallstab behandelt, um noch intakte Zellen aufzuschließen. Zur Abtrennung der Zellbestandteile und Fett wurde die Suspension 30 Minuten lang bei 4°C zentrifugiert, das Homogenat abdekantiert und mit einer Spur Thimerosal sterilisiert. Aus 5 Spiegelkarpfen wurden 260,7 ml Enzymlösung der Darmschleimhaut gewonnen, die Lösung wurde bei 4°C dunkel gelagert.

### b) Durchführung der in vitro Verdauungsuntersuchungen

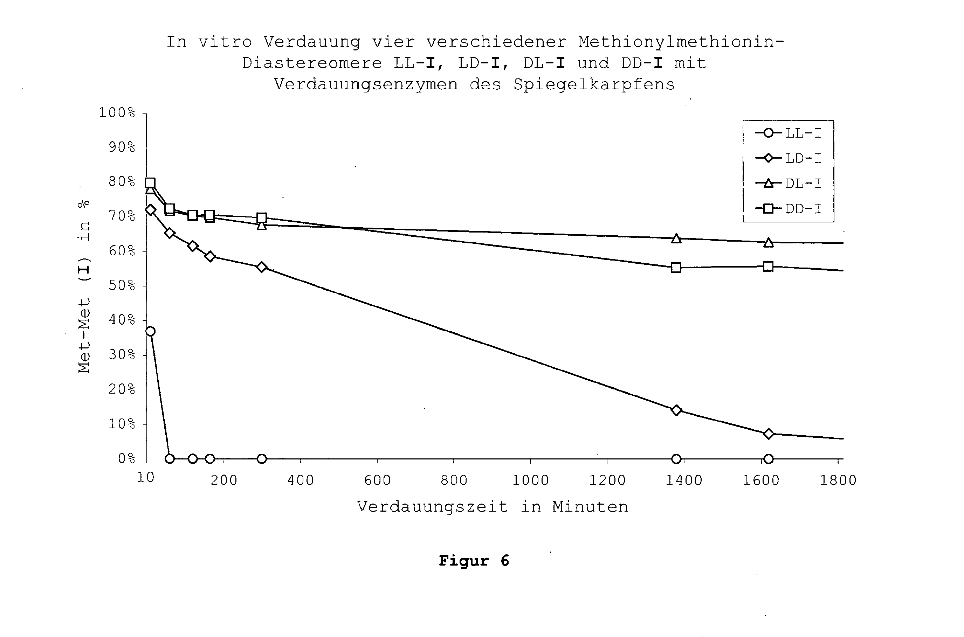

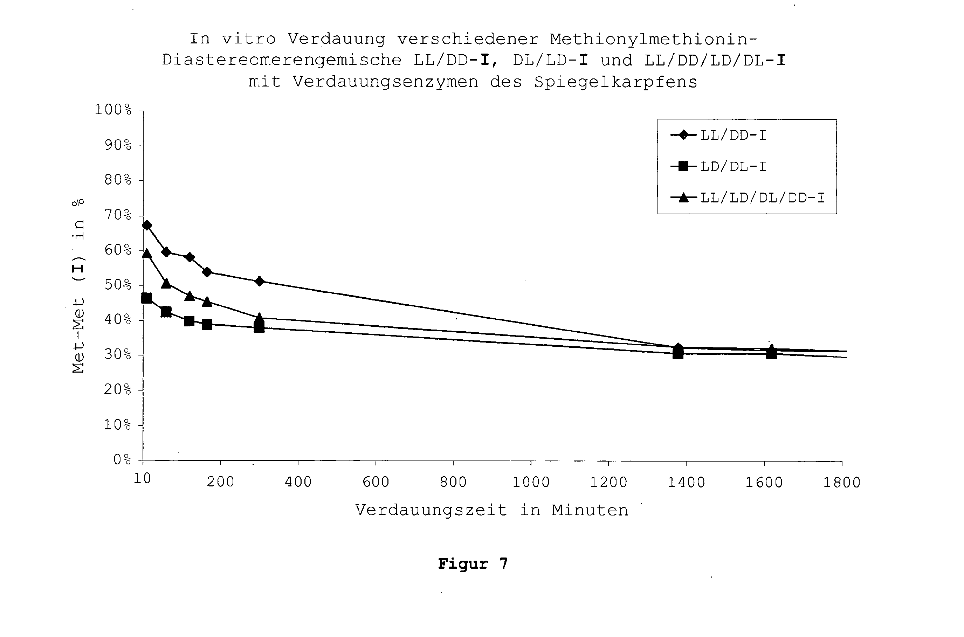

DL-Methionyl-DL-methionin (**I**) bzw. die entsprechenden Diastereomerenpaare DD/LL-**I** und DL/LD-**I** wurden in TRIS/HCl-Pufferlösung aufgenommen und mit der Enzymlösung versetzt. Als Vergleich und zur Abschätzung der rein chemischen Spaltungsgeschwindigkeit wurde jeweils ein Blindwert ohne Enzymlösung angesetzt. Von Zeit zu Zeit wurde eine Probe entnommen und deren Zusammensetzung mit Hilfe eines kalibriert ten HPLCs detektiert und quantifiziert. Der Umsatz wurde als Quotient der Fläche von Methionin und der Fläche von Methionylmethionin (**I**) bestimmt (siehe Figur 6 und 7).

**Tabelle 1**

| | | **Probe** | **Blindwert** |
|---|---|---|---|
| Vorlage | Substrat Met-Met (**I**) | 0,143 mmol (40,1 mg) | 0,143 mmol (40,1 mg) |
| | TRIS/HCl-Pufferlösung, pH 9,5 | 5,7 ml | 8,3 ml |
| Reaktionsstart | Enzymlösung ( 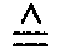 5% Karpfenlsg.) | 2,6 ml | --- |
| Reaktion | | 37 °C | 37 °C |

| | | | |
|---|---|---|---|
| Reaktionsabbr. 0,2 ml Reaktionslösung wurde in 9,8 ml 10%iger H₃PO₄-Lösung aufgenommen. | | | |

### Beispiel 23:

### In vitro Verdauungsversuche von DL-Methionyl-DL-methionin (I) mit Verdauungsenzymen aus karnivoren Forellen

### a) Isolierung der Verdäuungsenzyme aus Regenbogenforellen (Oncorhynchus mykiss)

Die Isolierung der Verdauungsenzyme wurde in Anlehnung an die Methode von EID und MATTY (Aquaculture 1989, 79, 111-119) durchgeführt. Dazu wurde der Darm von sechs einjährigen Regenbogenforellen (*Oncorhynchus mykiss)* freigelegt und wie in Beispiel 22 beschrieben aufgearbeitet.

### b) Durchführung der in vitro Verdauungsuntersuchungen

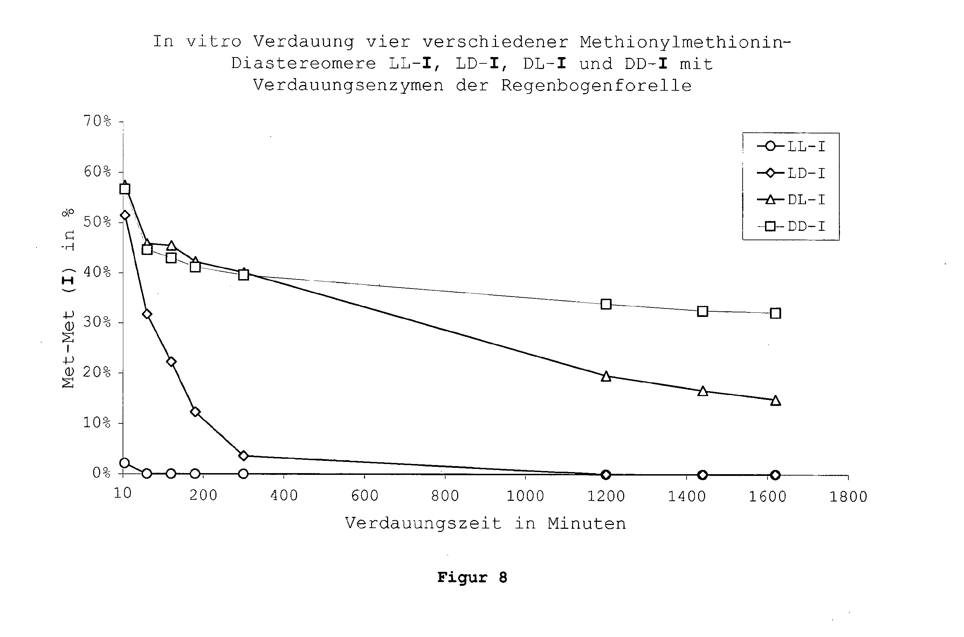

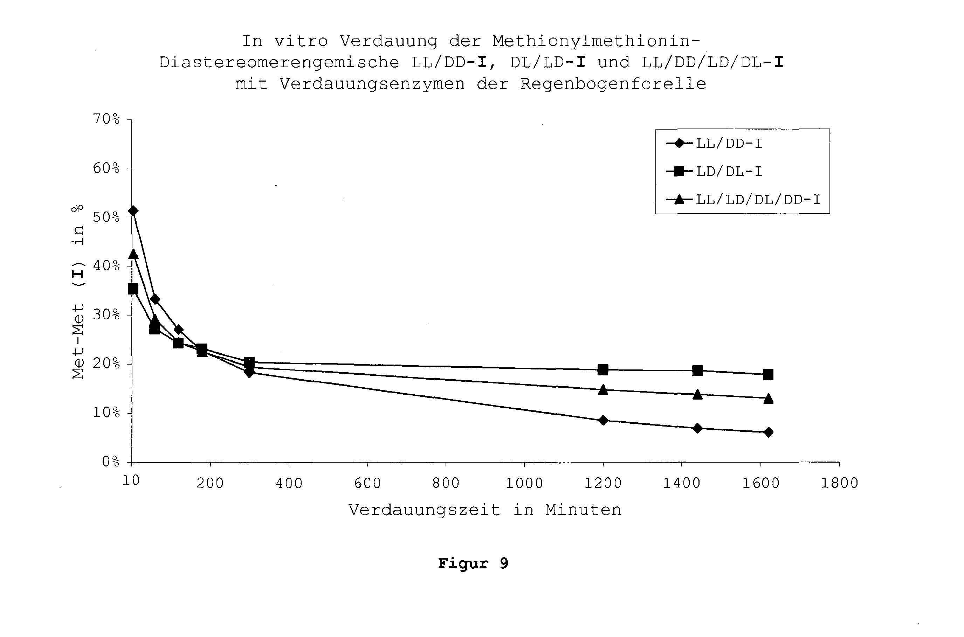

Die *in vitro* Untersuchungen wurden analog zu Beispiel 22 durchgeführt (siehe Figur 8 und 9).

**Tabelle 2**

| | | **Probe** | **Blindwert** |
|---|---|---|---|
| Vorlage | Substrat Met-Met(**I**) | 0,143 mmol (40,1 mg) | 0,143 mmol (40,1 mg) |
| | TRIS/HCl- Pufferlösung, pH 9,5 | 5,7 ml | 9,8 ml |
| Reaktionsstart | Enzymlösung ( 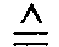 10% Forellelsg.) | 4,2 ml | --- |
| Reaktion | | 37 °C | 37 °C |

| | | | |
|---|---|---|---|
| Reaktionsabbr. 0,2 ml Reaktionslösung wurde in 9,8 ml 10%iger H₃PO₄-Lösung aufgenommen. | | | |

### Beispiel 24:

### In vitro Verdauungsversuche von DL-Methionyl-DL-methionin (I) mit Verdauungsenzymen aus omnivoren Shrimps

### a) Isolierung der Verdauungsenzyme aus Whiteleg Shrimps (Litopenaeus Vannamei)

Die Isolierung der Verdauungsenzyme wurde in Anlehnung an die Methode von Ezquerra und Garcia-Carreno (J. Food Biochem. 1999, 23, 59-74) durchgeführt. Dazu wurde das Hepatopankreas aus fünf Kilogramm Whiteleg Shrimps (*Litopenaeus Vannamei*) entfernt und zusammen mit zerstoßenem Eis mit einem Mixer zerkleinert. Die weitere Aufarbeitung wurde analog Beispiel 22 durchgeführt.

### b) Durchführung der in vitro Verdauungsuntersuchungen

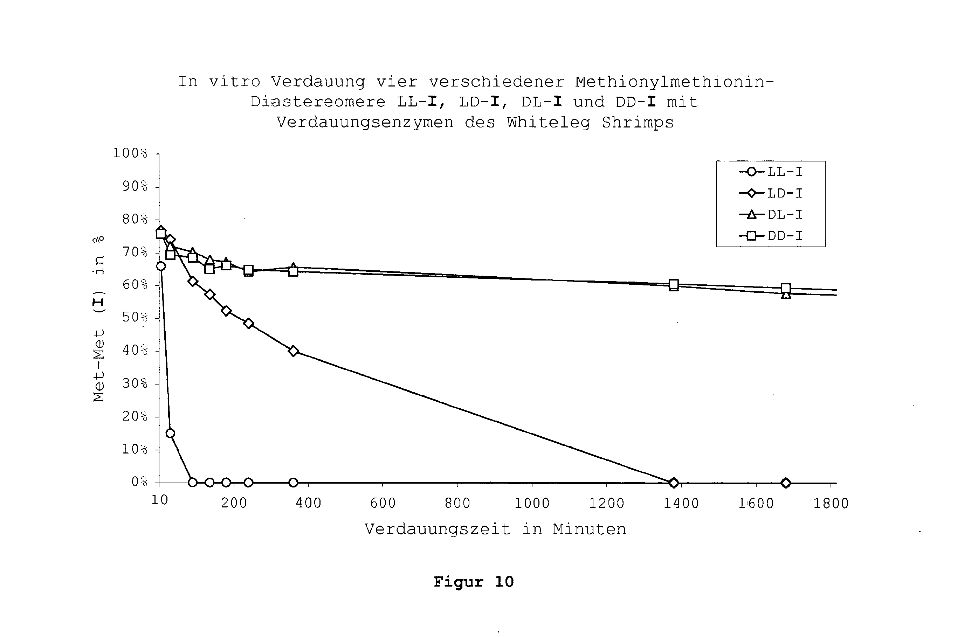

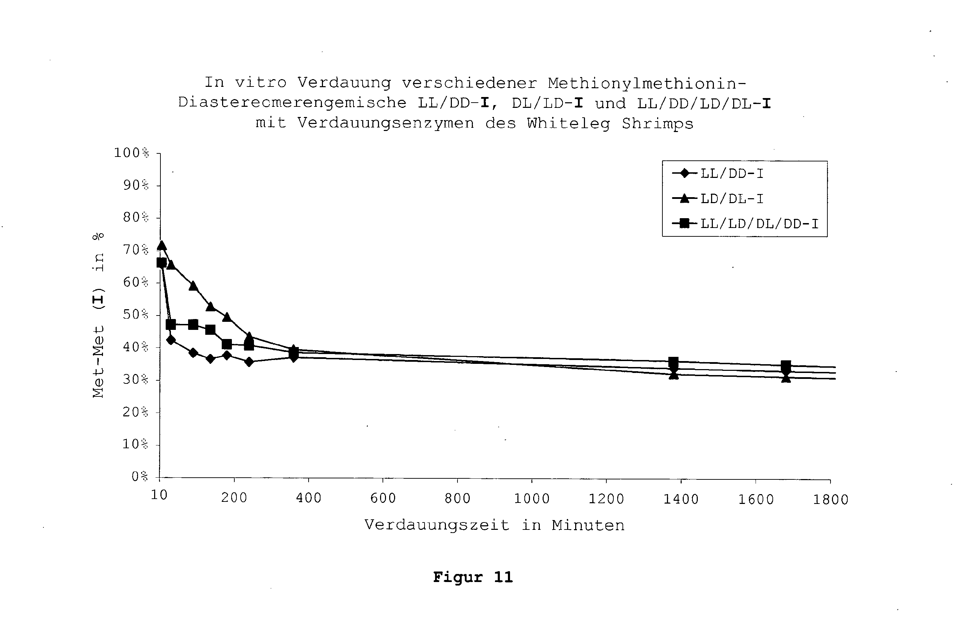

Die *in vitro* Untersuchungen wurden analog zu Beispiel 22 durchgeführt (siehe Figur 10 und 11).

**Tabelle 3**

| | | **Probe** | **Blindwert** |
|---|---|---|---|
| Vorlage | Substrat Met-Met (**I**) | 0,143 mmol (40, 1 mg) | 0,143 mmol (40,1 mg) |
| | TRIS/HCl-Pufferlösung, pH 9,5 | 5,7 ml | 7,9 ml |
| Reaktionsstart | Enzymlösung ( 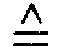 2 Shrimps) | 2,2 ml | --- |
| Reaktion | | 37 °C | 37 °C |

| | | | |
|---|---|---|---|
| Reaktionsabbr. 0,2 ml Reaktionslösung wurde in 9,8 ml 10%iger H₃PO₄-Lösung aufgenommen. | | | |

### Beispiel 25:

### Biotransformation von D- zu L-Methionin mit Enzymen aus Darm, Leber und Pankreas aus Spiegelkarpfen

### a) Isolierung der Verdauungsenzyme aus Spiegelkarpfen (Cyprinus carpio morpha noblis)

Die Isolierung der Verdauungsenzyme wurde in Anlehnung an die Methode von EID und MATTY (Aquaculture 1989, 79, 111-119) durchgeführt. Dazu wurde der Darm von fünf einjährigen Spiegelkarpfen(*Cyprinus carpio morpha noblis*) freigelegt und wie in Beispiel 22 beschrieben aufgearbeitet. Zur Isolation von Leberenzymen wurden die Lebern isoliert, homogenisiert und analog der Verarbeitung der Darmenzyme in Beispiel 22 behandelt. Analog dazu wurde auch mit der Enzymisolation aus der Pankreas verfahren.

### b) Durchführung der in vitro Biotransformation von D- zu L-Methionin

D-Methionin wurde in Pufferlösung aufgenommen und mit der Enzymlösung versetzt. Als Vergleich und zur Abschätzung der rein chemischen Umwändlungsgeschwindigkeit wurde jeweils ein Blindwert ohne Enzymlösung angesetzt. Nach 24 Stunden wurde eine Probe entnommen und die Zusammensetzung mit Hilfe eines kalibrierten HPLCs detektiert und quantifiziert. Der Umsatz wurde als Quotient der Fläche von L-Methionin und der Fläche von D-Methionin bestimmt (siehe Figur 4).

**Tabelle 4**

| | | **Probe** | **Blindwert** |
|---|---|---|---|
| Vorlage | Substrat D-Methionin | 0,143 mmol (21,3 mg) | 0,143 mmol (21,3 mg) |
| | Pufferlösung | 11,7 ml | 23,4 ml |
| Reaktionsstart | Enzymcocktail ( 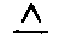 5% Karpfenlsg.) | 11,7 ml | --- |
| Reaktion | | 37 °C | 37 °C |

| | | | |
|---|---|---|---|
| Reaktionsabbr. 0,2 ml Reaktionslösung wurde in 9,8 ml 10%iger H₃PO₄-Lösung aufgenommen. | | | |

### Pufferlösungen:

| | |
|---|---|
| Citrat-Puffer: | pH 5, pH 6 und pH 7 |
| Phosphat-Puffer: | pH 8 |
| TRIS/HCl-Puffer: | pH 9 |

Enzymcocktail aus Darm-, Leber- und Pankreasenzymen ( 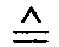 5% Karpfenlösung) :
2,6 ml Enzymlösung aus Darmschleimhaut
3,5 ml Enzymlösung aus Leber
5,6 ml Enzymlösung aus Pankreas

### Beispiel 26:

### Leaching-Verhalten der Diastereomerengemische von Methionylmethionin LL/DD/LD/DL-I, DD/LL-I bzw. DL/LD-I aus Futterpellets im Vergleich zu DL-Methionin, MHA und Calcium-MHA

### Futtermischung:

Als Futtermittelmatrix wurde eine Methionin defiziente Futtermischung aus gängigen Zutaten wie z.B. Sojaschrot, Sojaöl, Maisstärke, Weizenmehl, Fischmehl, Cellulose, kristalline essentielle Aminosäuren und Mineralien und Vitamine als Premixe verwendet. Diese Mischung wurde anschließend batchweise in 20 kg Ansätzen jeweils mit den in der Tabelle 5 aufgeführten Methioninderivaten mit 0,25% Supplementierungsrate (bezogen auf chwefeläquivalente) supplementiert, homogenisiert und anschließend unter Dampfbehandlung pelletiert. Als Vergleich zu Methionylmethionin (**I**) wurde je ein Pelletierungsversuch mit DL-Methionin, MHA (Methionin Hydroxy Analoges) und Calcium-MHA gemacht. Als Kontrollversuch wurde zudem eine Pelletierung ohne Zugabe eines Methioninderivats durchgeführt (siehe Tabelle 5).

**Tabelle 5**

| Nr. | Methioninderivat | Reinheit (wt%) | Molmasse (Monomer) | Einwaage |
|---|---|---|---|---|
| 1 | ohne Additiv | - | - | 0,00 g |
| 2 | DL-Methionin | 99,0% | 149,21 | 50,61 g |
| 3 | MHA | 88,0% | 150,19 | 57,14 g |
| 4 | Calcium-MHA (MHA-Ca) | 93,3% | 169,22 | 60,77 g |
| 5 | DD/LL/DL/LD-Metionylmethionin (**I**) | 99,7% | 140,20 | 47,13 g |

Während des gesamten Pelletierungsprozesses und Dampfbehandlung blieben alle Diastereomere von Methionylmethionin (**I**) stabil (siehe Tabelle 6).

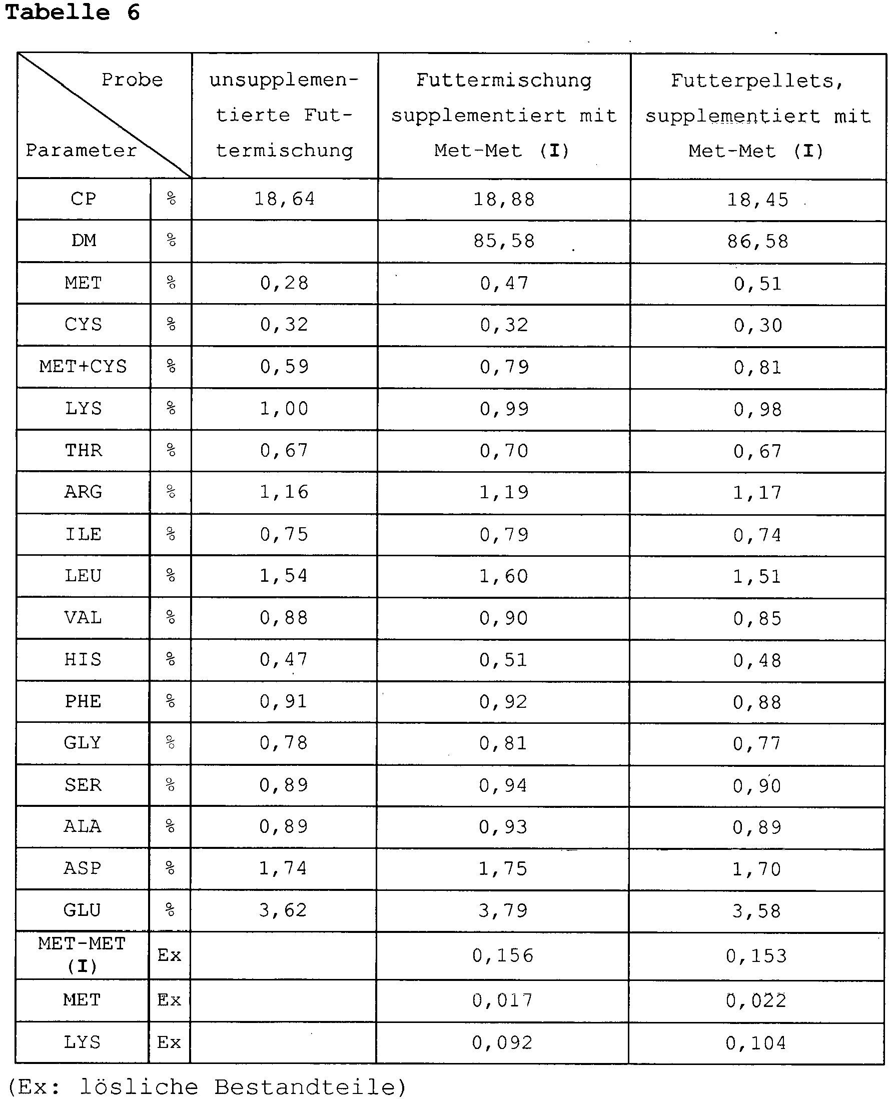

Dabei wurde die Aminosäurenbestimmung in Anlehnung an die EU-Methode 98/64/EC durchgeführt. Nach Extraktion der freien Aminosäuren und Methionylmethionin (**I**) wurden diese anschließend mit Hilfe eines Aminosäureanalysators durch Nachsäulenderivatisierung mit Ninhydrin bestimmt (siehe Tabelle 6).

Anschließend wurde das Leachingverhalten der Diastereomere von Methionylmethionin (**I**) aus den Futterpellets unter Wasser untersucht. Dabei wurde das Herauslösen von Methionylmethionin unter Wasser in Abhängigkeit der Zeit, Temperatur, Wasserzusammensetzung (Salz- oder Süßwasser) bestimmt. Dazu wurden 20,0 g der Futterpellets in einen engmaschigen Siebbeutel gegeben und in einen Erlenmeyerkolben mit 200 g Wasser vollständig eingetaucht. Anschließend wurde gesamte Erlenmeyerkolben bei einer konstanten Temperatur von 20°C mit einem Laborschüttelgerät kontinuierlich bewegt. In definierten Zeitabständen wurde dann jeweils eine Wasserprobe entnommen und der Gehalt der einzelnen Diastereomerenpaare von Methionylmethionin im Wasser mittels HPLC bestimmt (siehe Tabelle 7).

**Tabelle 7**

| Zeit | Methionin | MHA | MHA-Ca | LL/DD-**I** | DL/LD-**I** | LL/DD/LD/DL-**I** |
|---|---|---|---|---|---|---|
| 0 | 4,0% | 6,0% | 8,6% | 2,7% | 0,6% | 1,5% |
| 5 | 12,0% | 12,8% | 16,5% | 3,7% | 0,7% | 2,0% |
| 10 | 16,0% | 20,8% | 28,2% | 6,5% | 0,9% | 3,2% |
| 15 | 24,0% | 28,8% | 39,4% | 7,7% | 0,6% | 3,6% |
| 30 | 39,9% | 50,5% | 61,7% | 12,1% | 0,6% | 5,4% |
| 60 | 59,9% | 75,4% | 82,4% | 20,6% | 1,7% | 9,5% |
| 120 | 79,8% | 94,1% | 94,1% | 27,4% | 1,7% | 12,3% |
| 210 | 87,8% | 99,9% | 97,0% | 35,9% | 3,8% | 17,0% |

Als Vergleich wurden jeweils die Futterpellets, die mit DL-Methionin, MHA bzw. Calcium-MHA supplementiert waren, unter den gleichen Bedingungen untersucht und damit deren Leachingverhalten unter Wasser unter den jeweiligen Bedingungen bestimmt (siehe Figur 5 und Tabelle 7).

## Patentansprüche

1. Verwendung von DL-Methionyl-DL-methionin und dessen Salze als Futtermitteladditiv in Futtermittelmischungen für in Aquakulturen gehaltene Tiere, wobei als Salz bevorzugt werden Kationen aus der Gruppe der Alkali- und Erdalklimetalle, sowie Ammonium, Cu²⁺, Zn²⁺ und Co²⁺.

2. Verwendung nach Anspruch 1, wobei die Futtermittelmischung 0,01 bis 5 Gew.-%, bevorzugt 0,05 bis 0,5 Gew.-% DL-Methionyl-DL-methionin enthält.

3. Verwendung nach einem der Ansprüche 1 oder 2, wobei die Futtermittelmischung Protein und Kohlehydrate enthält, vorzugsweise auf Basis von Fisch-, Soja- oder Maismehl, und mit essentiellen Aminosäuren, Proteinen, Peptiden, Vitaminen, Mineralien, Kohlenhydraten, Fetten und Ölen supplementiert sein kann.

4. Verwendung nach einem der Ansprüche 1 bis 3, wobei DL-Methionyl-DL-methionin allein oder als Salz als DD/LL/LD/DL-Mischung, als DL/LD- oder DD/LL-Mischung vorliegt, bevorzugt jeweils zusätzlich in Mischung mit DL-Methionin, vorzugsweise mit einem DL-Methioninanteil von 0,01 bis 20 Gew.-%, besonders bevorzugt von 1 bis 10 Gew.-%.

5. Verwendung nach einem der Ansprüche 1 bis 4, wobei DL-Methionyl-DL-methionin als Enantiomerenpaar DL/LD-Methionylmethionin vorliegt.

6. Verwendung nach einem der Ansprüche 1 bis 5, wobei die in Aquakulturen gehaltene Tiere Süß- und Salzwasserfische und -krustentiere sind, vorzugsweise ausgewählt aus der Gruppe bestehend aus Karpfen, Forellen, Lachse, Welse, Barsche, Plattfische, Störe, Thunfische, Aale, Brassen, Dorsche, Shrimps, Krill und Prawns, ganz besonders für Silver- (*Hypophthalmichthys molitrix*), Gras- (*Ctenopharyngodon idella*), Schuppen-(*Cyprinus carpio*) und Bigheadkarpfen (*Aristichthys nobilis*), Karausche (*Carassius carassius*), Catla (*Catla Catla*), Roho Labeo (*Labeo rohita*), pazifischer und atlantischer Lachs (*Salmon salar* und *Oncorhynchus kisutch*), Regenbogenforelle *(Oncorhynchus mykiss),* amerikanischer Wels (*Ictalurus punctatus*), afrikanischer Wels (*Clarias gariepinus*), Pangasius (*Pangasius bocourti* und *Pangasius hypothalamus*), Niltilapie (*Oreochromis niloticus*), Milchfisch (*Chanos chanos*), Cobia (*Rachycentron canadum*), Whiteleg Shrimp (*Litopenaeus vannamei*), Black Tiger Shrimp (*Penaeus monodon*) und Giant River Prawn (*Macrobrachium rosenbergii*).

7. Verfahren zur Herstellung von DL-Methiohyl-DL-methionin (**I**) mit der Formel 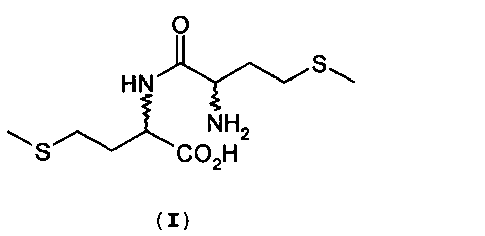 unter Umsetzung eines Harnstoffderivates der allgemeinen Formel **II**, 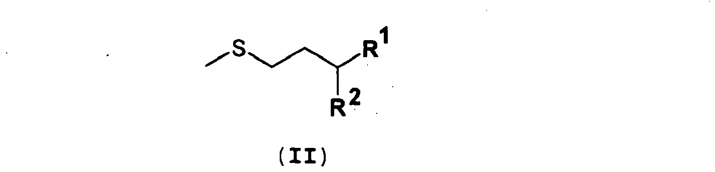 wobei die Reste R¹ und R² in den Harnstoffderivaten **IIa, IIb, IIc, IIe und IIf** wie folgt definiert sind:
wobei
**IIa: R¹** = COOH, **R²** = NHCONH₂
**IIb: R¹** = CONH₂, **R²** = NHCONH₂
**IIc : R¹** = CONH₂, **R²** = NH₂
**IIe: R¹** = CN, **R²** = OH
**IIf : R¹** = CN, **R² =** NH₂
bedeutet,
zu DL-Methionyl-DL-methionin.

8. Verfahren nach Anspruch 7 wobei der pH-Wert der das Harnstoffderivat enthaltenden Lösung auf 8 bis 14, besonders bevorzugt auf 10 bis 13 eingestellt wird.

9. Das Verfahren nach Anspruch 7 oder 8, wobei die Umsetzung bei einer Temperatur von 50 bis 200°C, bevorzugt bei einer Temperatur von 80 bis 170°C und besonders bevorzugt bei einer Temperatur von 130 bis 160 °C erfolgt und/oder die Umsetzung unter Druck, bevorzugt bei einem Druck von 3 bis 20 bar, besonders bevorzugt bei einem Druck von 6 bis 15 bar durchgeführt wird.

10. Das Verfahren nach Anspruch 7 umfassend die folgenden Schritte:
a) Umsetzung des Harnstoffderivates gemäß den Formeln **IIa, IIb, IIc, IIe und IIf** zu einem Diketopiperazin (III) der Formel 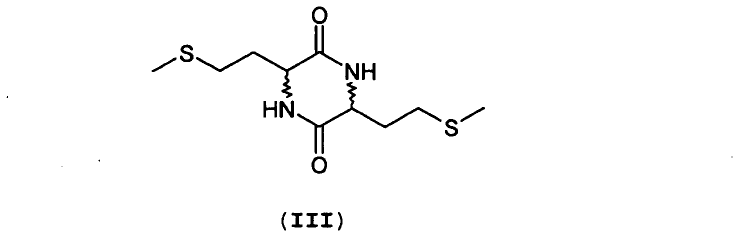
b) Umsetzung des Diketopiperazins (**III**) zum DL-Methionyl-DL-methionin (**I**)
und dabei vorzugsweise die Umsetzung des Harnstoffderivates zum Diketopiperazin bei einer Temperatur von 50°C bis 200°C, bevorzugt von 100°C bis 180°C und besonders bevorzugt von 140°C bis 170°C durchgeführt wird.

11. Das Verfahren nach Anspruch 10, wobei die Umsetzung des Harnstoffderivates zum Diketopiperazin unter Druck, bevorzugt bei einem Druck von 3 bis 20 bar, besonders bevorzugt bei einem Druck von 6 bis 15 bar durchgeführt wird.

12. Das Verfahren nach einem der Ansprüche 10 oder 11, wobei die Umsetzung des Harnstoffderivates zum Diketopiperazin in Gegenwart einer Base erfolgt und die Base dabei vorzugweise ausgewählt ist aus der Gruppe Stickstoff haltiger Basen, NH₄HCO₃, (NH₄)₂CO₃, KHCO₃, K₂CO₃, NH₄OH/CO₂-Mischung, Carbamatsalze, Alkali- und Erdalkalibasen.

13. Das Verfahren nach einem der Ansprüche 10 bis 12, wobei die Umsetzung des Harnstoffderivates zum Diketopiperazin durch Reaktion mit Methionin erfolgt.

14. Das Verfahren nach einem der Ansprüche 10 bis 13, wobei die Umsetzung des Diketopiperazin zum DL-Methionyl-DL-methionin durch saure Hydrolyse erfolgt und vorzugsweise dabei die saure Hydrolyse in Gegenwart einer Säure erfolgt, die ausgewählt ist aus der Gruppe der Mineralsäuren, HCl, H₂CO₃, CO₂/H₂O, H₂SO₄, Phosphorsäuren, Carbonsäuren und Hydroxycarbonsäuren.

15. Das Verfahren nach einem der Ansprüche 10 bis 13, wobei die Umsetzung des Diketopiperazin zum DL-Methionyl-DL-methionin durch basische Hydrolyse erfolgt und vorzugsweise dabei die basische Hydrolyse bei einem pH von 7 bis 14, besonders bevorzugt bei einem pH von 9 bis 12, ganz besonders bevorzugt bei einem pH von 10 bis 11 durchgeführt wird, um DL-Methionyl-DL-methionin zu erhalten.

16. Das Verfahren nach Anspruch 15, wobei die basischen Bedingungen unter Verwendung einer Substanz eingestellt werden ausgewählt aus der Gruppe Stickstoff haltiger Basen, NH₄HCO₃, (NH₄) ₂CO₃, NH₄OH/CO₂-Mischung, Carbamatsalze, KHCO₃, K₂CO₃, Carbonate, Alkalibasen und Erdalkalibasen.

17. Das Verfahren nach einem der Ansprüche 14 bis 16, wobei die Umsetzung bei einer Temperatur von 50°C bis 200°C, vorzugsweise 80°C bis 180°C und besonders bevorzugt von 90°C bis 160°C durchgeführt wird.

18. Das Verfahren nach einem der Ansprüche 10 bis 13, wobei die Umsetzung des Diketopiperazin zum DL-Methionyl-DL-methionin durch Einleiten von CO₂ in eine basische Lösung, vorzugsweise in eine basische Ammonium-, Kalium- oder Natriumhydroxidlösung erfolgt.

19. Das Verfahren nach einem der Ansprüche 10 bis 18, wobei das Diketopiperazin vor der Hydrolyse isoliert wird und dabei das Diketopiperazin vorzugsweise durch Kristallisation aus der Reaktionslösung, bevorzugt bei einer Temperatur von -30 bis 120°C, besonders bevorzugt bei einer Temperatur von 10 bis 70°C isoliert wird.

20. Verfahren zur Isolation des Diastereomerengemisches aus DD/LL/DL/LD-Methionylmethionin durch Kristallisation aus basischen Reaktionslösungen, die nach irgendeinem der Ansprüche 7 bis 13 und 15 bis 19 erhalten wurden.

21. Das Verfahren nach Anspruch 20, wobei die Lösung mit einer Säure auf einen pH-Wert von 5 bis 9, bevorzugt auf einen pH-Wert von 5 bis 7, vorzugsweise auf einen pH-Wert von ca. 5,6 eingestellt wird.

22. Das Verfahren nach Anspruch 20 oder 21, wobei die Kristallisation in Gegenwart einer Säure erfolgt, ausgewählt aus der Gruppe der Mineralsäuren, HCl, H₂CO₃, CO₂/H₂O, H₂SO₄, Phosphorsäuren, Carbonsäuren, Hydroxycarbonsäuren.

23. Verfahren zur Isolation des Diastereomerengemisches aus DD/LL/DL/LD-Methionylmethionin durch Kristallisation aus sauren Reaktionslösungen, die nach Anspruch 14 erhalten wurden und dabei die Lösung mit einer Base auf einen pH-Wert von 5 bis 9, bevorzugt auf einen pH-Wert von 5 bis 7, besonders bevorzugt auf einen pH-Wert von ca. 5,6 eingestellt wird.

24. Verfahren nach Anspruch 23, wobei die Base ausgewählt ist aus der Gruppe NH₄HCO₃, (NH₄)₂CO₃, Stickstoff haltige Basen, NH₄OH, Carbamatsalze, KHCO₃, K₂CO₃, Carbonate, Alkali- und Erdalkalibasen.

## Claims

1. Use of DL-methionyl-DL-methionine and salts thereof as feed additive in feed mixtures for animals kept in aquacultures, where preference is given as salt to cations from the group of alkali metals and alkaline earth metals, and ammonium, Cu²⁺_{,} Zn²⁺ and Co²⁺.

2. Use according to Claim 1, where the feed mixture comprises from 0.01 to 5% by weight, preferably 0.05 to 0.5% by weight, of DL-methionyl-DL-methionine.

3. Use according to either of Claims 1 and 2, where the feed mixture comprises protein and carbohydrates, preferably based on fish meal, soybean meal or corn meal, and may be supplemented with essential amino acids, proteins, peptides, vitamins, minerals, carbohydrates, fats and oils.

4. Use according to any of Claims 1 to 3, where DL-methionyl-DL-methionine is present alone or as salt as DD/LL/LD/DL mixture, as DL/LD or DD/LL mixture, preferably in each case additionally mixed with DL-methionine, preferably with a DL-methionine content of from 0.01 to 20% by weight, particularly preferably from 1 to 10% by weight.

5. Use according to any of Claims 1 to 4, where DL-methionyl-DL-methionine is present as a DL/LD-methionylmethionine pair of enantiomers.

6. Use according to any of Claims 1 to 5, where the animals kept in aquacultures are salt and fresh water fish and crustaceans, preferably selected from the group consisting of carp, trout, salmon, catfish, perch, flatfish, sturgeon, tuna, eels, bream, cod, shrimps, krill and prawns, very preferably for silver carp (*Hypophthalmichthys molitrix*), grass carp (*Ctenopharyngodon idella*), common carp (*Cyprinus carpio*) and bighead carp (*Aristichthys nobilis*), carassius (*Carassius carassius*), catla (*Catla Catla*), Roho labeo (*Labeo rohita*), Pacific and Atlantic salmon (*Salmon salar* and *Oncorhynchus kisutch*), rainbow trout *(Oncorhynchus mykiss),* American catfish (*Ictalurus punctatus*), African catfish (*Clarias gariepinus*), pangasius (*Pangasius bocourti* and *Pangasius hypothalamus*), Nile tilapia (*Oreochromis niloticus*), milkfish (*Chanos chanos*), cobia (*Rachycentron canadum*), whiteleg shrimp (*Litopenaeus vannamei*), black tiger shrimp (*Penaeus monodon*) and giant river prawn *(Macrobrachium rosenbergii).*

7. Process for preparing DL-methionyl-DL-methionine (**I**) with the formula 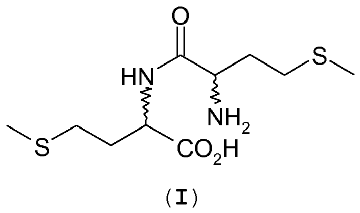 by reacting a urea derivative of the general formula **II** 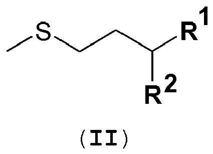 where the radicals R¹ and R² in the urea derivatives **IIa, IIb, IIc, IIe and IIf** are defined as follows:
where
**IIa: R¹** = COOH, **R²** = NHCONH₂
**IIb: R¹** = CONH₂, **R²** = NHCONH₂
**IIc: R¹** = CONH₂, **R² =** NH₂
**IIe : R¹** = CN, **R²** = OH
**IIf: R¹** = CN, **R²** = NH₂
to give DL-methionyl-DL-methionine.

8. Process according to Claim 7, where the pH of the solution comprising the urea derivative is adjusted to from 8 to 14, particularly preferably to from 10 to 13.

9. Process according to Claim 7 or 8, where the reaction takes place at a temperature of from 50 to 200°C, preferably at a temperature of from 80 to 170°C and particularly preferably at a temperature of from 130 to 160°C and/or the reaction is carried out under pressure, preferably under a pressure of from 3 to 20 bar, particularly preferably under a pressure of from 6 to 15 bar.

10. Process according to Claim 7, comprising the following steps:
a) reaction of the urea derivative of formulae **IIa, IIb, IIc, IIe** and **IIf** to give a diketopiperazine (**III**) of the formula 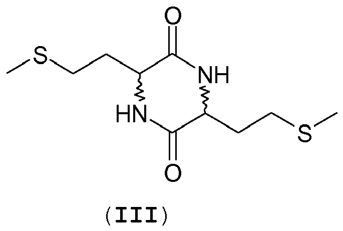
b) reaction of the diketopiperazine (**III**) to give DL-methionyl-DL-methionine (**I**) and preferably the reaction of the urea derivative to give the diketopiperazine is carried out at a temperature of from 50°C to 200°C, preferably from 100°C to 180°C and particularly preferably from 140°C to 170°C.

11. Process according to Claim 10, where the reaction of the urea derivative to give the diketopiperazine is carried out under pressure, preferably under a pressure of from 3 to 20 bar, particularly preferably under a pressure of from 6 to 15 bar.

12. Process according to either of Claims 10 and 11, where the reaction of the urea derivative to give the diketopiperazine takes place in the presence of a base and the base is preferably selected from the group of nitrogen-containing bases, NH₄HCO₃, (NH₄) ₂CO₃, KHCO₃, K₂CO₃, NH₄OH/CO₂ mixture, carbamate salts, alkali metal and alkaline earth metal bases.

13. Process according to any of Claims 10 to 12, where the reaction of the urea derivative to give the diketopiperazine takes place by reaction with methionine.

14. Process according to any of Claims 10 to 13, where the reaction of the diketopiperazine to give DL-methionyl-DL-methionine takes place by acidic hydrolysis and preferably the acidic hydrolysis takes place in the presence of an acid which is selected from the group of mineral acids, HCl, H₂CO₃, CO₂/H₂O, H₂SO₄, phosphoric acids, carboxylic acids and hydroxy carboxylic acids.

15. Process according to any of Claims 10 to 13, where the reaction of the diketopiperazine to give DL-methionyl-DL-methionine takes place by basic hydrolysis and preferably the basic hydrolysis is carried out at a pH of from 7 to 14, particularly preferably at a pH of from 9 to 12, very particularly preferably at a pH of from 10 to 11, in order to obtain DL-methionyl-DL-methionine.

16. Process according to Claim 15, where the basic conditions are adjusted by using a substance selected from the group of nitrogen-containing bases, NH₄HCO₃, (NH₄) ₂CO₃, NH₄OH/CO₂ mixture, carbamate salts, KHCO₃, K₂CO₃, carbonates, alkali metal bases and alkaline earth metal bases.

17. Process according to any of Claims 14 to 16, where the reaction is carried out at a temperature of from 50°C to 200°C, preferably 80°C to 180°C and particularly preferably from 90°C to 160°C.

18. Process according to any of Claims 10 to 13, where the reaction of the diketopiperazine to give DL-methionyl-DL-methionine takes place by introducing CO₂ into a basic solution, preferably into a basic ammonium hydroxide, potassium hydroxide or sodium hydroxide solution.

19. Process according to any of Claims 10 to 18, where the diketopiperazine is isolated before the hydrolysis and the diketopiperazine is preferably isolated by crystallization from the reaction solution, preferably at a temperature of from -30 to 120°C, particularly preferably at a temperature of from 10 to 70°C.

20. Process for isolating the mixture of DD/LL/DL/LD-methionylmethionine diastereomers by crystallization from basic reaction solutions which have been obtained according to any of Claims 7 to 13 and 15 to 19.

21. Process according to Claim 20, where the solution is adjusted with an acid to a pH of from 5 to 9, preferably to a pH of from 5 to 7, preferably to a pH of about 5.6.

22. Process according to Claim 20 or 21, where the crystallization takes place in the presence of an acid selected from the group of mineral acids, HCl, H₂CO₃, CO₂/H₂O, H₂SO₄, phosphoric acids, carboxylic acids, hydroxy carboxylic acids.

23. Process for isolating the mixture of DD/LL/DL/LD-methionylmethionine diastereomers by crystallization from acidic reaction solutions obtained according to Claim 14 and the solution is adjusted with a base to a pH of from 5 to 9, preferably to a pH of from 5 to 7, particularly preferably to a pH of about 5.6.

24. Process according to Claim 23, where the base is selected from the group of NH₄HCO₃, (NH₄) ₂CO₃, nitrogen-containing bases, NH₄OH, carbamate salts, KHCO₃, K₂CO₃, carbonates, alkali metal and alkaline earth metal bases.

## Revendications

1. Utilisation de DL-méthionyl-DL-méthionine et de ses sels comme additif pour l'alimentation animale dans des mélanges pour l'alimentation animale destinés à des animaux élevés dans des aquacultures, en préférant comme sel des cations du groupe des métaux alcalins et alcalino-terreux, ainsi que l'ammonium, Cu²⁺, Zn²⁺ et Co²⁺.

2. Utilisation selon la revendication 1, le mélange pour l'alimentation animale contenant 0,01 à 5% en poids, de préférence 0,05 à 0,5% en poids de DL-méthionyl-DL-méthionine.

3. Utilisation selon l'une quelconque des revendications 1 ou 2, le mélange pour l'alimentation animale contenant des protéines et des hydrates de carbone, de préférence à base de farine de poisson, de soja ou de maïs et pouvant être additionné d'acides aminés essentiels, de protéines, de peptides, de vitamines, de minéraux, d'hydrates de carbone, de graisses et d'huiles.

4. Utilisation selon l'une quelconque des revendications 1 à 3, la DL-méthionyl-DL-méthionine se trouvant seule ou sous forme de sel sous forme de mélange DD/LL/LD/DL, de mélange DL/LD ou de mélange DD/LL, de préférence à chaque fois en plus en mélange avec de la DL-méthionine, de préférence avec une proportion de DL-méthionine de 0,01 à 20% en poids, de manière particulièrement préférée de 1 à 10% en poids.

5. Utilisation selon l'une quelconque des revendications 1 à 4, la DL-méthionyl-DL-méthionine se trouvant sous forme de paire énantiomère DL/LD-méthionylméthionine.

6. Utilisation selon l'une quelconque des revendications 1 à 5, les animaux élevés dans des aquacultures étant de poissons et des crustacés d'eau douce et d'eau de mer, de préférence choisis dans le groupe constitué par les carpes, les truites, les saumons, les silures, les perches, les poissons plats, les esturgeons, les thons, les anguilles, les brèmes, les cabillauds, les crevettes, les krills, tout particulièrement pour la carpe argentée (Hypophthalmichthys molitrix), la carpe de roseau (Ctenopharyngodon idella), la carpe commune (Cyprinus carpio) et la carpe marbrée (Aristichthys nobilis), le carassin (Carassius carassius), le catla (Catla Catla), le labéo roho (Labeo rohita), le saumon du pacifique et atlantique (Salmon salar et Oncorhynchus kisutch), la truite arc en ciel (Oncorhynchus mykiss), la silure américaine (Ictalurus punctatus), la silure africaine (Clarias gariepinus), le pangasius (Pangasius bocourti et Pangasius hypothalamus), le tilapia du Nil (Oreochromis niloticus), le poisson-lait (Chanos chanos), le cobia (Rachycentron canadum), la crevette à pattes blanches (Litopenaeus vannamei), la crevette géante tigrée (Penaeus monodon) et la crevette géante d'eau douce (Macrobrachium rosenbergii).

7. Procédé pour la préparation de DL-méthionyl-DL-méthionine (I) présentant la formule 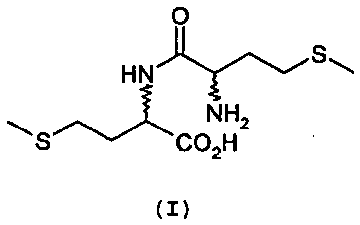 par transformation d'un dérivé d'urée de formule générale II, 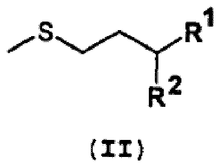 les radicaux R¹ et R² dans les dérivés d'urée IIa, IIb, IIc, IIe et IIf étant définis comme suit :
où
**IIa: R¹** = COOH, **R²** = NHCONH₂
**IIb: R¹** = CONH₂, **R²** = NHCONH₂
**IIc: R¹** = CONH₂, **R²** = NH₂
**IIe: R¹** = CN, **R²** = OH
**IIf: R¹** = CN, **R²** = NH₂
en DL-méthionyl-DL-méthionine.

8. Procédé selon la revendication 7, le pH de la solution contenant le dérivé d'urée étant réglé à 8 jusqu'à 14, de manière particulièrement préférée à 10 jusqu'à 13.

9. Procédé selon la revendication 7 ou 8, la transformation ayant lieu à une température de 50 à 200°C, de préférence à une température de 80 à 170°C et de manière particulièrement préférée à une température de 130 à 160°C et/ou la transformation étant réalisée sous pression, de préférence à une pression de 3 à 20 bars, de manière particulièrement préférée à une pression de 6 à 15 bars.

10. Procédé selon la revendication 7, comprenant les étapes suivantes :
a) transformation du dérivé d'urée selon les revendications IIa, IIb, IIc, IIe et IIf en une dicétopipérazine (III) de formule 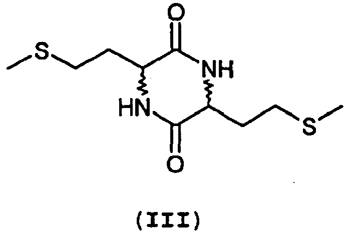
b) transformation de la dicétopipérazine (III) en DL-méthionyl-DL-méthionine (I)
et la transformation du dérivé d'urée en dicétopipérazine étant de préférence réalisée à une température de 50°C à 200°C, de préférence de 100°C à 180°C et de manière particulièrement préférée de 140°C à 170°C.

11. Procédé selon la revendication 10, la transformation du dérivé d'urée en dicétopipérazine étant réalisée sous pression, de préférence à une pression de 3 à 20 bars, de manière particulièrement préférée à une pression de 6 à 15 bars.

12. Procédé selon l'une quelconque des revendications 10 ou 11, la transformation du dérivé d'urée en dicétopipérazine ayant lieu en présence d'une base et la base étant de préférence choisie dans le groupe des bases azotées, NH₄HCO₃, (NH₄) ₂CO₃, KHCO₃, K₂CO₃, mélange NH₄OH/CO₂, les sels de carbamate, les bases de métal alcalin et alcalino-terreux.

13. Procédé selon l'une quelconque des revendications 10 à 12, la transformation du dérivé d'urée en dicétopipérazine ayant lieu par réaction avec de la méthionine.

14. Procédé selon l'une quelconque des revendications 10 à 13, la transformation de la dicétopipérazine en DL-méthionyl-DL-méthionine ayant lieu par hydrolyse acide et l'hydrolyse acide ayant de préférence lieu en présence d'un acide, choisi dans le groupe des acides minéraux, HCl, H₂CO₃, CO₂/H₂O, H₂SO₄, acides phosphoriques, acides carboxyliques et acides hydroxycarboxyliques.

15. Procédé selon l'une quelconque des revendications 10 à 13, la transformation de la dicétopipérazine en DL-méthionyl-DL-méthionine ayant lieu par hydrolyse basique et l'hydrolyse basique étant de préférence réalisée à un pH de 7 à 14, de manière particulièrement préférée à un pH de 9 à 12, de manière tout particulièrement préférée à un pH de 10 à 11, pour obtenir la DL-méthionyl-DL-méthionine.

16. Procédé selon l'une quelconque des revendications 15, les conditions basiques étant réglées en utilisant une substance, choisie dans le groupe des bases azotées, NH₄HCO₃, (NH₄) ₂CO₃, mélange NH₄0H/CO₂, sels de carbamate, KHCO₃, K₂CO₃, carbonates, bases alcalines et bases alcalino-terreuses.

17. Procédé selon l'une quelconque des revendications 14 à 16, la transformation étant réalisée à une température de 50°C à 200°C, de préférence de 80°C à 180°C et de manière particulièrement préférée de 90°C à 160°C.

18. Procédé selon l'une quelconque des revendications 10 à 13, la transformation de la dicétopipérazine en DL-méthionyl-DL-méthionine ayant lieu en faisant passer du CO₂ dans une solution basique, de préférence dans une solution basique d'hydroxyde d'ammonium, de potassium ou de sodium.

19. Procédé selon l'une quelconque des revendications 10 à 18, la dicétopipérazine étant isolée avant l'hydrolyse et la dicétopipérazine étant de préférence isolée par cristallisation à partir de la solution réactionnelle, de préférence à une température de -30 à 120°C, de manière particulièrement préférée à une température de 10 à 70°C.

20. Procédé pour l'isolement du mélange de diastéréo-isomères à partir de DD/LL/DL/LD-méthionylméthionine par cristallisation à partir de solutions réactionnelles qui ont été obtenues selon l'une quelconque des revendications 7 à 13 et 15 à 19.

21. Procédé selon la revendication 20, la solution étant réglée avec un acide à un pH de 5 à 9, de préférence à un pH de 5 à 7, de préférence à un pH d'environ 5,6.

22. Procédé selon la revendication 20 ou 21, la cristallisation ayant lieu en présence d'un acide, choisi dans le groupe des acides minéraux HCl, H₂CO₃, CO₂/H₂O, H₂SO₄, acides phosphoriques, acides carboxyliques, acides hydroxycarboxyliques.

23. Procédé pour l'isolement du mélange de diastéréo-isomères à partir de DD/LL/DL/LD-méthionylméthionine par cristallisation à partir de solutions réactionnelles acides qui ont été obtenues selon la revendication 14 et en réglant la solution avec une base à un pH de 5 à 9, de préférence à un pH de 5 à 7, de manière particulièrement préférée à un pH d'environ 5,6.

24. Procédé selon la revendication 23, la base étant choisie dans le groupe NH₄HCO₃, (NH₄) ₂CO₃, bases azotées, NH₄OH, sels de carbamate, KHCO₃, K₂CO₃, carbonates, bases de métal alcalin et alcalino-terreux.
